(19) Europäisches Patentamt
European Patent Office
Office européen des brevets

(11) EP 4 606 824 A1

(12) **EUROPEAN PATENT APPLICATION**
published in accordance with Art. 153(4) EPC

(43) Date of publication:
**27.08.2025 Bulletin 2025/35**

(21) Application number: **23879870.6**

(22) Date of filing: **19.10.2023**

(51) International Patent Classification (IPC):
***C08F 2/50*** *(2006.01)* ***A61K 6/62*** *(2020.01)*
***A61K 8/37*** *(2006.01)* ***A61Q 3/02*** *(2006.01)*
***B33Y 70/00*** *(2020.01)* ***B33Y 80/00*** *(2015.01)*
***C08G 18/30*** *(2006.01)* ***C08G 18/42*** *(2006.01)*
***C08G 18/60*** *(2006.01)* ***C09D 11/30*** *(2014.01)*

(52) Cooperative Patent Classification (CPC):
**A61K 6/62; A61K 8/37; A61Q 3/02; B33Y 70/00; B33Y 80/00; C08F 2/50; C08G 18/30; C08G 18/42; C08G 18/60; C09D 11/30**

(86) International application number:
**PCT/JP2023/037865**

(87) International publication number:
**WO 2024/085227 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2022 JP 2022169434**

(71) Applicant: **KJ Chemicals Corporation**
**Tokyo 103-0023 (JP)**

(72) Inventors:
- **TAKEDA, Yoshimi**
**Yatsushiro-shi Kumamoto 866-0081 (JP)**
- **KIYOSADA, Toshitsugu**
**Yatsushiro-shi Kumamoto 866-0081 (JP)**

(74) Representative: **ABG Intellectual Property Law, S.L.**
**Avenida de Burgos, 16D**
**Edificio Euromor**
**28036 Madrid (ES)**

# (54) PHOTOPOLYMERIZATION INITIATOR

(57) An object of the present invention is to provide a photopolymerization initiator that is hardly affected by inhibition by oxygen, has good photoinitiating property in air, has high sensitivity to long-wavelength light, and can suppress odor and bleed-out problems of a cured product to be obtained.

A photopolymerization initiator having one or more benzophenone groups and one or more saturated or unsaturated 5 or more-membered cyclic substituents having heteroatoms in a molecule, wherein one or more saturated or unsaturated 5 or more-membered cyclic substituents having heteroatoms are bonded to one or more carbon atoms of an aryl group of one or more benzophenone groups through a carboxylic acid ester group or a carboxylic acid amide group.

EP 4 606 824 A1

## Description

### TECHNICAL FIELD

The present disclosure relates to a photopolymerization initiator.

### BACKGROUND

A photo-curing reaction using active energy rays such as visible light or ultraviolet (UV) rays is a reaction in which: a curable composition containing a photopolymerization initiator is irradiated with UV to generate active species such as radicals; a compound having unsaturated groups is polymerized; and the liquid composition is solidified (cured) in a short time; and is used in a wide range of fields such as in paints and coating agents, pressure-sensitive adhesive agents, adhesives, elastomerbased materials, inkjet inks, materials for sealing, sealant materials, dental hygiene materials, and photosensitive materials. In particular, since the photocurable resin can be cured in any place and in any shape, the photo-curing reaction is widely used in the field of nail cosmetics such as gel nails and the field of three-dimensional stereolithography materials.

The photopolymerization initiator can be classified into an intramolecular cleavage type and a hydrogen abstraction type according to a radical generation mechanism after absorbing light. The former is a type that cleaves in a molecule to generate a radical, and the latter is a type that abstracts hydrogen from a hydrogen donor to generate a radical. In the intramolecular cleavage type, since decomposition products derived from the initiator remain in a cured product, problems such as deterioration in durability of the cured product, generation of odor, and discoloration over time occur, and there is also a problem of low safety. In the hydrogen abstraction type, since there is no decomposition product of the initiator, it has been increasingly receiving attention in recent years. Studies have been actively conducted to improve the efficiency of photopolymerization initiation and to use additives such as a hydrogen donor, a photosensitizer, and a curing accelerator in combination.

### SUMMARY OF THE INVENTION

### PROBLEMS TO BE SOLVED BY THE INVENTION

An object of the present disclosure is to provide a hydrogen abstraction type photopolymerization initiator which has high radical generating ability and high reactivity of generated radicals, is hardly inhibited from oxygen, can be used with long-wavelength (360 nm to 420 nm) light, has good compatibility with general-purpose monomers and oligomers, and has excellent yellowing resistance.

### SOLUTION TO THE PROBLEMS

As a result of extensive research, the present inventors have found a photopolymerization initiator having one or more benzophenone groups and one or more saturated or unsaturated 5 or more-membered cyclic substituents having heteroatoms in a molecule, wherein one or more saturated or unsaturated 5 or more-membered cyclic substituents having heteroatoms are bonded to one or more carbon atoms of an aryl group of one or more benzophenone groups through a carboxylic acid ester group or a carboxylic acid amide group, and have achieved the object.

### EFFECTS OF THE INVENTION

The photopolymerization initiator of the present disclosure has high initiation efficiency with respect to a long-wavelength light having a wavelength of 360 nm to 420 nm and a single-wavelength light output from a UV-LED light source such as 365 nm, 385 nm, 395 nm, and 405 nm, is hardly inhibited from curing by oxygen even in an air atmosphere, does not generate decomposition products in a photoinitiation reaction and a photopolymerization reaction (curing), and has high safety. In addition, the photopolymerization initiator has good compatibility with general-purpose monomers and oligomers, and the curable composition containing the photopolymerization initiator has excellent transparency. The cured product obtained by curing the curable composition has very little odor, bleed-out, yellowing over time, deterioration, and the like, and has high durability and safety. The photopolymerization initiator of the present disclosure can be suitably used for various applications using active energy ray curable compositions such as an ink composition, a pressure-sensitive adhesive composition, an adhesive composition, a coating composition, a sealant composition, an inkjet ink, a three-dimensional modeling ink, a nail cosmetic composition, a dental material composition, and a photosensitive composition.

DESCRIPTION OF EMBODIMENTS

Hereinafter, embodiments of the present disclosure are described in detail, but the scope of the present invention is not limited to the embodiments described herein, and various modifications can be made without departing from the purpose of the present invention. In addition, in a case where a plurality of upper limit values and lower limit values are described for a specific parameter, any upper limit value and any lower limit value among these upper limit values and lower limit values can be combined to form a suitable numerical range.

An embodiment of the present disclosure is a photopolymerization initiator (A) having one or more benzophenone groups and one or more saturated or unsaturated 5 or more-membered cyclic substituents having heteroatoms in a molecule, wherein one or more saturated or unsaturated 5 or more-membered cyclic substituents having heteroatoms are bonded to one or more carbon atoms of an aryl group of one or more benzophenone groups through a carboxylic acid ester group or a carboxylic acid amide group.

The photopolymerization initiator (A) has one or more benzophenone groups and one or more saturated or unsaturated 5 or more-membered cyclic substituents having heteroatoms (hereinafter also referred to as heterocycle) in a molecule, and one or more heterocycles are linked to carbon atoms of an aryl group of the benzophenone groups through a carboxylic acid ester group or a carboxylic acid amide group. The benzophenone group is a hydrogen abstraction type photopolymerization initiating functional group, and the heterocycle is a hydrogen donor. The photopolymerization initiator (A) contains the benzophenone group and the heterocycle, and efficiently occurs hydrogen abstraction intramolecular and/or intermolecular upon irradiation with active energy rays. As a result, a sufficient photoinitiation effect can be obtained without adding alcohols, amines, or the like as a general-purpose hydrogen donor. The inventors presume the reason as follows. 1) Since the electron density of a heteroatom is high and the activity of the hydrogen atoms around the heteroatom is high, hydrogen is easily abstracted from the heterocycle. 2) Since hydrogen atoms bonded to a heteroatom and/or hydrogen atoms bonded to a carbon atom adjacent to a heteroatom are all donor sources, and the photopolymerization initiator (A) has cyclic structures, the number of highly active hydrogen atoms around the heteroatom is large. 3) Since a heteroatom can easily absorb a peroxide radical generated from oxygen, the influence of oxygen is suppressed by the presence of the heterocycle.

In addition, it has been confirmed that the presence of a carboxylic acid ester group or a carboxylic acid amide group between the benzophenone group and the heterocycle improves the compatibility between the hydrophobic benzophenone group and the hydrophilic heterocycle, and the photoinitiation effect tends to be further enhanced.

An embodiment of the present disclosure is a photopolymerization initiator (A) having one or more ethylenically unsaturated groups of one or more kinds selected from one or more (meth)acrylamide groups, (meth)acrylate groups, vinyl groups, vinyl ether groups, alkyl vinyl ether groups, allyl groups, (meth)allyl ether groups, styryl groups, and maleimide groups in a molecule. Due to the presence of the ethylenically unsaturated group(s), the photopolymerization initiator (A) is incorporated into the structure of the cured product by a covalent bond after photopolymerization reaction and does not bleed-out over time, and the resulting cured product is improved in durability, yellowing resistance, moisture resistance, and the like. When the photopolymerization initiator (A) has two or more ethylenically unsaturated groups, they may be the same or different. In addition, the (meth)acrylamide group, the (meth)acrylate group, and the allyl group have high curability, and can be cured at high speed even with a light source of a long-wavelength light or a single-wavelength light, which is preferable.

An embodiment of the present disclosure is a photopolymerization initiator (A) having one or more 5- or larger-membered cyclic substituents containing a heteroatom (heterocycles), and such cyclic substituents are one or more groups selected from piperidine group, pyrrolidine group, piperazine group, pyridine group, morpholine group, tetrahydrofuran group, hydrofuran group, crown ether group and tetrahydrothiopyran group. These substituents show high effect of suppressing oxygen inhibition, and the photopolymerization initiator (A) containing these substituents can cause the polymerization initiation reaction with high efficiency even in air, which is preferable. In addition, it is more preferable to have a morpholine group, a tetrahydrofuran group, or a piperidine group, which is highly effective as a hydrogen donating group. These heterocycles can be contained alone or in combination of two or more.

An embodiment of the present disclosure is a photopolymerization initiator (A) further having one or more groups selected from urethane group, urea group, ester group, thioester group, amide group and imide group in a molecule. These groups have heteroatoms, and hydrogen atoms bonded to a heteroatom and/or hydrogen atoms bonded to a carbon atom adjacent to a heteroatom are all hydrogen donors, thereby improving the initiation efficiency of the photopolymerization initiator (A). At the same time, these groups have an effect of suppressing oxygen inhibition, and the curability of the curable composition is improved even in air. In addition, from the viewpoint of industrial convenience, the photopolymerization initiator (A) preferably contains a urethane group, a urea group, an ester group, an amide group, or an imide group. These groups can be contained alone or in combination of two or more.

The photopolymerization initiator (A) of the present disclosure can be obtained by a reaction of a carboxylic acid and/or a carboxylic anhydride having a benzophenone group (hereinafter also referred to as benzophenone-based compound (a1)) with a compound having a heterocycle and having functional groups which can react with carboxylic acid

and/or carboxylic anhydride (hereinafter also referred to as heterocyclic compound (a2)). Examples of the functional group capable of reacting with a carboxylic acid and/or a carboxylic anhydride include hydroxyl group, amine group, epoxy group, oxazoline group, carbodiimide group, isocyanate group, thiol group, phenol group, and halogen group. From the viewpoint of easily reacting at normal temperature (0°C to 150°C) and normal pressure (0.8 to 1.2 atm), hydroxyl group, amine group, epoxy group, oxazoline group, isocyanate group, thiol group, and halogen group are preferable. From the viewpoint of being able to use either the direct reaction method or the indirect reaction method of (a1) and (a2), it is more preferable that the heterocyclic compound (a2) has at least one hydroxyl group, amine group or epoxy group, and the reaction method of (a1) and (a2) will be described below. Regarding (a2), these groups can be contained alone or in combination of two or more.

Methods for producing the photopolymerization initiator (A) by directly reacting the benzophenone compound (a1) and the heterocyclic compound (a2) include a method in which (a1) and (a2) are mixed at once and reacted, and a method in which one of (a1) or (a2) is dropped into the other and reacted sequentially. Methods of indirectly reacting (a1) with (a2), include a method in which (a1) reacts with a compound (a5) capable of reacting with (a1) to obtain a compound (a3) having benzophenone group and having reactive functional groups (hereinafter also referred to as reactive groups) such as hydroxyl group, carboxylic acid group, amine group, epoxy group, oxazoline group, isocyanate group, thiol group, or halogen group has been introduced, and then (a3) reacts with a heterocyclic compound (a2), and a method in which (a2) reacts with a compound (a5) capable of reacting with (a2) to obtain a compound (a4) having a heterocycle and having reactive groups such as hydroxyl group, carboxylic acid group, amine group, epoxy group, oxazoline group, isocyanate group, thiol group, or halogen group, and then (a4) reacts with the benzophenone-based compound (a1).

The reaction for producing the photopolymerization initiator (A) can appropriately proceed in a temperature range of 0°C to 150°C, and a solvent, a catalyst, and other additives may be used as necessary. In order to suppress radical generation during production, it is preferable to perform the reaction in an environment in which active energy rays are blocked, and it is more preferable to perform the reaction in a dark room, a yellow room in which active energy rays having a wavelength of 500 nm or less are cut, or a red safe light.

Examples of the benzophenone-based compound (a1) include benzophenone-2-carboxylic acid, 4-methylbenzophenone-3'-carboxylic acid, 4-phenylbenzophenone-2'-carboxylic acid, 4-methoxybenzophenone-4'-carboxylic acid, 4,4'-benzophenone dicarboxylic acid, 3,4-benzophenone dicarboxylic acid, 2,3'-dimethyl-4,4'-benzophenone dicarboxylic acid, 2,5,4'-benzophenone tricarboxylic acid, 3,3',4,4'-benzophenone tetracarboxylic acid, 2,2'-dimethyl-3,3',4,4'-benzophenone tetracarboxylic acid, 3,3',4,4'-benzophenone tetracarboxylic acid dianhydrate, 2,2'-dimethyl-3,3',4,4'-benzophenone tetracarboxylic acid dianhydrate, and 5-methyl-3,3',4,4'-benzophenone tetracarboxylic acid dianhydrate. Regarding (a1), these can be used alone or in combination of two or more. Among them, since an acid anhydride has high reactivity with the heterocyclic compound (a2), (a1) is preferably 3',4,4'-benzophenone tetracarboxylic acid dianhydrate, 2,2'-dimethyl-3,3',4,4'-benzophenone tetracarboxylic acid dianhydrate, or 5-methyl-3,3',4,4'-benzophenone tetracarboxylic acid dianhydrate, and is more preferably 3,3',4,4'-benzophenone tetracarboxylic acid dianhydrate from the viewpoint of easy availability of industrial products.

Examples of the heterocyclic compound (a2) include tetrahydrofurfuryl alcohol, 3-hydroxytetrahydrofuran, (S)-(+)-2,2-dimethyl-1,3-dioxolane-4-methanol, glycerol 1,2-carbonate, tetrahydro-4-pyranol, 2-(hydroxymethyl)-12-crown 4-ether, N-hydroxysuccinimide, 1-(2-hydroxyethyl)-2-pyrrolidone, 2-(2-hydroxyethyl)-1-methylpyridine, 1-piperidine ethanol, 4-methylpiperazine-1-ethanol, tetrahydro-2H-thiopyran-4-ol, 4-(2-hydroxyethyl)-morpholine, 4-(3-hydroxyethyl)-morpholine, N-(2-hydroxypropyl)-morpholine, N-(2-hydroxyethyl)maleimide, tetrahydrofurfurylamine, 3-(aminomethyl)tetrahydrofuran, 2-(aminomethyl)-1,3-dioxolane, 5-aminopyrimidine, 4-(1-pyrrolidinyl)piperidine, 1-aminopiperidine, 1-(3-aminopropyl)-2-methylpiperidine, 1-(2-methoxyethyl)piperazine, 1-(2-pyrimidyl)piperazine, 2-(furfurylthio) ethylamine, morpholine, thiomorpholine, 4-aminomorpholine, 4-(2-aminoethyl)morpholine, and 4-morpholinoaniline. Among them, from the viewpoint of high stability of a cyclic substituent, 4-hydroxy-1-methylpiperidine, 1-piperidine ethanol, 4-methylpiperazine-1-ethanol, 4-(2-hydroxyethyl)-morpholine, 4-(3-hydroxyethyl)-morpholine, N-(2-hydroxypropyl)-morpholine, 1-aminopiperidine, 1-(3-aminopropyl)-2-methylpiperidine, 1-(2-methoxyethyl)piperazine, 4-(4-methyl-1-piperazinyl)aniline, morpholine, 4-aminomorpholine, 4-(2-aminoethyl)morpholine, and 4-morpholinoaniline are more preferable. Regarding (a2), these can be used alone or in combination of two or more.

The compound (a5) capable of reacting with (a1) or (a2) is not particularly limited as long as it is a compound containing a reactive group such as a hydroxyl group, a carboxylic acid group, an amine group, an epoxy group, an oxazoline group, an isocyanate group, a thiol group or a halogen group, or an ethylenically unsaturated group. Examples of the compound (a5) include water, linear alcohols having 1 to 24 carbon atoms or branched or alicyclic alcohols having 3 to 24 carbon atoms having hydroxyl groups; linear alkylene glycols having 2 to 24 carbon atoms or branched or alicyclic alkylene glycols having 3 to 24 carbon atoms; (meth)acrylates containing hydroxyl groups; (meth)acrylamides containing hydroxyl groups; linear alkylenediamine having 2 to 24 carbon atoms or branched or alicyclic alkylenediamine having 3 to 24 carbon atoms having amino groups; phenylenediamine; 1,2-butylene oxide having epoxy groups; 1,2-epoxydodecane; 1,2-epoxytetradecane; butyl glycidyl ether; glycidyl phenyl ether; 2-ethylhexyl glycidyl ether; dodecyl glycidyl ether; glycidyl (meth)acrylate; 4-hydroxybutyl acrylate glycidyl ether; 1,2-epoxycyclohexane; and 1,2-epoxy-4-butyl-4-octanol.

In addition, since an epoxy group reacts with a carboxylic acid to generate a hydroxyl group, and urethanization or esterification can be performed using the hydroxyl group, (a5) is preferably a compound having epoxy groups. Further, butyl glycidyl ether, glycidyl phenyl ether, 2-ethylhexyl glycidyl ether, dodecyl glycidyl ether, 2-[(butoxymethoxy)methyl] oxirane, 1,2-epoxydodecane, 1,2-epoxytetradecane, glycidyl (meth)acrylate, and 4-hydroxybutyl acrylate glycidyl ether are more preferable from the viewpoint of high reactivity. The compounds (a5) can be used alone or in combination of two or more.

Examples of the solvent used for producing the photopolymerization initiator (A) of the present disclosure include hydrocarbon-based solvents such as toluene, xylene, n-hexane, and cyclohexanone; ketone-based solvents such as acetone, methyl ethyl ketone, and methyl isobutyl ketone; ester-based solvents such as ethyl acetate and butyl acetate; halogenated hydrocarbon solvents such as methylene chloride and chlorobenzene; and polar solvents having a high boiling point such as N,N'-dimethylformamide, 3-methoxy-N,N'-dimethylpropionamide, 3-butoxy-N,N'-dimethylpropionamide, dimethylacetamide, dimethylsulfoxide, 2-pyrrolidone, N-methyl-2-pyrrolidone, and 1,3-dimethyl-2-imidazolidinone. In addition, a monofunctional or polyfunctional monomer and/or oligomer reactive with the photopolymerization initiator (A) and the raw materials (a1) and (a2) thereof can also be used as a solvent of the reaction. For example, various (meth)acrylic acid esters having a chain and/or cyclic hydrocarbon group (1 to 22 carbon atoms) or an alkoxy group (1 to 22 carbon atoms), N-substituted (meth)acrylamide, and N,N-disubstituted (meth)acrylamide, and (meth)acryloylmorpholine (meth)acryloyl, N-vinylpyrrolidone, and the like are also used.

In the production of the photopolymerization initiator (A) of the present disclosure, the direct reaction and various indirect reactions can proceed without using a catalyst. In addition, by using a catalyst, both the direct reaction and the indirect reaction can proceed at a lower temperature or at a higher speed, which is preferable. Examples of the catalyst used for the production of (A) include thionyl chloride, a quaternary ammonium salt, a tertiary phosphine derivative, a tertiary amine derivative, and an organometallic compound. Examples of the quaternary ammonium salt include tetrabutylammonium bromide, triethylbenzylammonium chloride, tetrabutylphosphonium bromide, and tetraphenylphosphonium bromide. Examples of the tertiary phosphine include triarylphosphines such as triphenylphosphine and tritolylphosphine; tricycloalkylphosphines such as tricyclohexylphosphine; and trialkylphosphines such as triethylphosphine. Examples of the tertiary amine include trialkyl (1 to 8 carbon atoms) amines such as triethylamine, tributylamine, and dimethylethylamine; and dialkyl (1 to 8 carbon atoms) arylamines such as diethylbenzylamine such as dimethylbenzylamine. Examples of the organometallic compound include metal salts of metals such as zinc, tin, lead, zirconium, bismuth, cobalt, manganese, and iron with organic acids such as octenoic acid and naphthenic acid; metal chelate compounds such as dibutyltin dilaurate, dioctyltin dilaurate, tin 2-ethylhexanoate, dibutyltin diacetylacetonate, zirconium tetraacetylacetonate, titanium acetylacetonate, acetylacetone aluminum, acetylacetone cobalt, acetylacetone iron, acetylacetone copper, and acetylacetone zinc; potassium or sodium salts of alkyl (1 to 8 carbon atoms) phosphonic acid; and sodium or potassium salts of fatty acids having 8 to 20 carbon atoms. Among them, a quaternary ammonium salt, a tertiary amine derivative, a tertiary phosphine derivative, and a tin-based, zirconium-based, or iron-based organometallic compound, which have a high catalytic effect, are more preferable. These catalysts can be used alone or in combination of two or more.

The amount of the catalyst used for producing the photopolymerization initiator (A) is not particularly limited. It is preferably 0.001 to 5.0% by mass ratio with respect to the total mass of each raw material. When the amount is 0.001% or more, the reaction can proceed quickly, and when the amount is 5.0% or less, coloring by the catalyst can be suppressed, which is preferable. Furthermore, the amount is more preferably 0.01 to 1.0%.

The method for introducing an ethylenically unsaturated group into the molecule of the photopolymerization initiator (A) is not particularly limited. Examples thereof include using a benzophenone-based compound (a1) containing an ethylenically unsaturated group and/or a heterocyclic compound (a2) containing an ethylenically unsaturated group, and using a compound (a5) containing an ethylenically unsaturated group capable of reacting with (a1) and/or (a2). The compound (a5) containing an ethylenically unsaturated group has a structure formed by any combination of one or more reactive groups selected from the group consisting of a hydroxyl group, a carboxylic acid group, an amine group, an epoxy group, an oxazoline group, an isocyanate group, a thiol group, and a halogen group and one or more ethylenically unsaturated groups selected from the group consisting of a (meth)acrylamide group, a (meth)acrylate group, a vinyl group, a vinyl ether group, an alkyl vinyl ether group, an allyl group, a (meth)allyl ether group, a styryl group, and a maleimide group. Examples of the compound (a5) include a compound having a hydroxyl group and a (meth)acrylate group, a compound having a hydroxyl group and a (meth)acrylamide group, a compound having a hydroxyl group and a vinyl group, a compound having a hydroxyl group and an allyl group, a compound having a hydroxyl group and a maleimide group, a compound having an amino group and a (meth)acrylate group, a compound having an amino group and a (meth) acrylamide group, a compound having an amino group and a vinyl group, a compound having an amino group and an allyl group, a compound having an amino group and a maleimide group, a compound having a carboxyl group and a (meth) acrylate group, a compound having a carboxyl group and a (meth)acrylamide group, a compound having a carboxyl group and a vinyl group, a compound having a carboxyl group and an allyl group, and a compound having a carboxyl group and a maleimide group. These (a1), (a2), and (a5) can be each independently used alone or in combination of two or more.

Specific examples of the compound (a5) include hydroxyalkyl (1 to 22 carbon atoms) (meth)acrylates such as hydroxyethyl (meth)acrylate, hydroxyisopropyl (meth)acrylate, and hydroxypropyl (meth)acrylate; N-hydroxyalkyl (1 to 22 carbon atoms) (meth)acrylamides such as N-hydroxymethyl (meth)acrylamide, N-hydroxyisopropyl (meth)acrylamide, and N,N-dihydroxyethyl (meth)acrylamide; N-alkyl (1 to 22 carbon atoms) hydroxyalkyl (1 to 22 carbon atoms) (meth) acrylamides such as N-methylhydroxyethyl (meth)acrylamide and N-ethylhydroxypropyl (meth)acrylamide; N,N-dihydroxyalkyl (1 to 22 carbon atoms) (meth)acrylamides such as N,N-dihydroxymethyl (meth)acrylamide and N,N-dihydroxyethyl (meth)acrylamide; hydroxyalkyl (1 to 22 carbon atoms) vinyls such as 3-buten-1-ol, 4-penten-1-ol, and 5-hexen-1-ol; hydroxyalkyl (1 to 22 carbon atoms) allyl; N-hydroxyalkyl (1 to 22 carbon atoms) maleimides such as N-hydroxymethyl maleimide and N-hydroxyethyl maleimide; hydroxyalkyl (1 to 22 carbon atoms) vinyl ethers such as ethylene glycol monovinyl ether and tetramethylene glycol monovinyl ether; N-aminoalkyl (1 to 22 carbon atoms) (meth) acrylamide; N-aminoalkyl (1 to 22 carbon atoms)-N-alkyl (1 to 22 carbon atoms) (meth)acrylamide; and N,N-diaminoalkyl (1 to 22 carbon atoms) (meth)acrylamide.

The method for introducing a urethane group, a urea group, an ester group, an amide group, and an imide group into the molecule of the photopolymerization initiator (A) is not particularly limited. Examples thereof include (1) a method for further reacting a compound or the like having one or more groups selected from a hydroxyl group, an amine group, a carboxylic acid group, and an isocyanate group (hereinafter also referred to as urethane group or the like-introduced compound) with reactive products of the benzophenone-based compound (a1) and the heterocyclic compound (a2); (2) a method for reacting the urethane group or the like-introduced compound with (a1) and then further reacting it with (a2); (3) a method for reacting the urethane group or the like-introduced compound with (a2) and then further reacting it with (a1); and (4) a method for simultaneously reacting the urethane group or the like-introduced compound with (a1) and (a2). Examples of the urethane group or the like-introduced compound include a monourethane group or the like-introduced compound having one group of any one kind selected from a hydroxyl group, an amine group, a carboxylic acid group, or an isocyanate group in a molecule, and a polyurethane group or the like-introduced compound having two or more groups of any one or more kinds selected from a hydroxyl group, an amine group, a carboxylic acid group, or an isocyanate group in a molecule. Examples of the polyurethane group or the like-introduced compound include a polyol, a polyamine, a polycarboxylic acid, a polyisocyanate, a polyamino acid, an amino group-containing polyol, a hydroxyl group-containing polyamine, and a hydroxyl group-containing polycarboxylic acid, and the polyurethane group or the like-introduced compound may have a polyester skeleton, a polyether skeleton, a polycarbonate skeleton, a polyolefin skeleton (a polyalkadiene skeleton and/or a hydrogenated polyalkadiene skeleton), a polyacrylic skeleton, and a silicone skeleton (various modified polydimethylsiloxanes). These urethane group or the like-introduced compounds can be used alone or in combination of two or more.

The molecular weight of the photopolymerization initiator (A) can be optionally adjusted by combining various raw materials, and is preferably 500 to 100,000 in number average. When the number average molecular weight is 500 or more, the content of a low-molecular-weight component having a molecular weight of less than 500 in a cured product obtained after photopolymerization reaction is low, so that the cured product has high safety, durability, heat resistance, and the like. When the number average molecular weight is 100,000 or less, the polarity (balance between hydrophilicity and hydrophobicity) of (A) can be easily adjusted, the solubility in a general-purpose monomer or oligomer used for an active energy ray curable composition is high, the viscosity of a curable composition containing (A) can be easily adjusted within a range suitable for various processing types such as application, injection, and extrusion, and the obtained curable composition and cured product have high transparency. Furthermore, depending on the molecular weight of (A), (A) can be classified into a low-molecular-weight type having a number average molecular weight of 500 to less than 1,000, a medium-molecular-weight type having a number average molecular weight of 1,000 to less than 10,000, and a high-molecular-weight type having a number average molecular weight of 10,000 to 100,000. The low-molecular-weight type (A) is mainly a compound in which a heterocycle is directly or indirectly bonded to a carboxylic acid ester group and/or a carboxylic acid amide group directly bonded to a benzophenone group represented by general formula (1). The medium-molecular-weight type (A) is mainly a compound in which a heterocycle is directly or indirectly bonded to a carboxylic acid ester group and/or a carboxylic acid amide group directly bonded to a benzophenone group represented by general formula (1), and at the same time, a structural unit derived from a urethane group or the like-introduced compound having one or more skeletons selected from a polyester skeleton, a polyether skeleton, a polycarbonate skeleton, a polyolefin skeleton, and a polyacrylic skeleton, a urethane group, and the like are bonded, and the order and position of bonding the heterocycle to the structural unit derived from the urethane group or the like-introduced compound, the urethane group, and the like are not limited. The high-molecular-weight type (A) is mainly a compound having a structure in which a heterocycle is directly or indirectly bonded to a carboxylic acid ester group and/or a carboxylic acid amide group directly bonded to a benzophenone group represented by general formula (1), and at the same time, the structure being formed by repeating bonds of a structural unit derived from a urethane group or the like-introduced compound having one or more skeletons selected from a polyester skeleton, a polyether skeleton, a polycarbonate skeleton, a polyolefin skeleton, and a polyacrylic skeleton, a urethane group, and the like, and the order and position of bonding the heterocycle to the repeating structural unit derived from the urethane group or the like-introduced compound, the urethane group, and the like are not

limited. Among them, the medium-molecular-weight type photopolymerization initiator is particularly preferable because it has good solubility in various general-purpose organic solvents, monomers, or oligomers used for the curable composition, the viscosity of the curable resin composition to be obtained can be appropriately adjusted, and it has high polymerization initiating property with respect to active energy rays, particularly high sensitivity to long-wavelength light having a wavelength of 360 nm to 420 nm while having excellent workability.

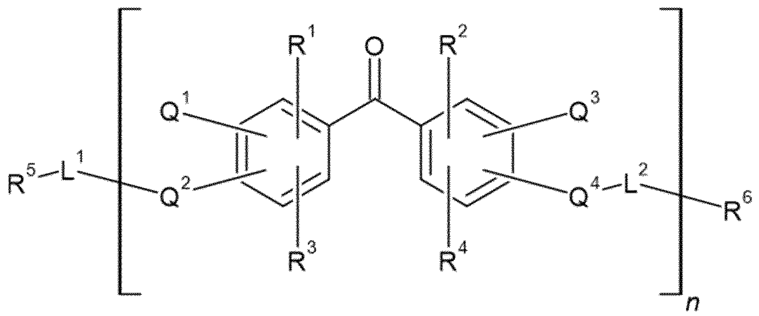

general formula (1)

wherein $Q^1$ and $Q^3$ are each independently a hydrogen atom or a monovalent organic group represented by general formula (2) or general formula (3), and any one or more of $Q^1$ and $Q^3$ are a monovalent organic group having one or more saturated or unsaturated 5 or more-membered cyclic substituents having heteroatoms,
$Q^2$ and $Q^4$ are each independently a divalent organic group represented by general formula (4) or general formula (5), $L^1$ and $L^2$ are each independently a direct bond or a divalent organic group containing one or more of a urethane group, a urea group, an ester group, an amide group, and an imide group, and $Q^2$-$L^1$-$R^5$ and $Q^4$-$L^2$-$R^6$ may be a hydrogen atom, except a case where $Q^2$-$L^1$-$R^5$ and $Q^4$-$L^2$-$R^6$ are simultaneously a hydrogen atom together with $Q^1$ and $Q^3$,

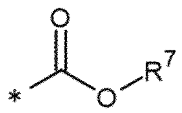

general formula (2)

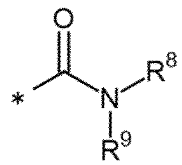

general formula (3)

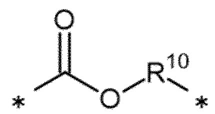

general formula (4)

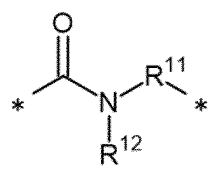

general formula (5)

$R^1$ to $R^9$ and $R^{12}$ each independently represent a hydrogen atom or a chain or cyclic, saturated or unsaturated monovalent hydrocarbon group having 1 to 36 carbon atoms in which one or more hydrogen atoms may be substituted with a hydroxyl group, an amine group, a thiol group, or a halogen group and one or more carbon atoms may be substituted with an ether group, an amino group, a thioether group, or a thioester group,
$R^{10}$ and $R^{11}$ each independently represent a direct bond or a chain or cyclic, saturated or unsaturated divalent hydrocarbon group having 1 to 36 carbon atoms in which one or more hydrogen atoms may be substituted with a hydroxyl group, an amine group, a thiol group, or a halogen group and one or more carbon atoms may be substituted with an ether group, an amino group, a thioether group, or a thioester group,
$R^8$ and $R^9$ may be taken together with a nitrogen atom carrying $R^8$ and $R^9$ to form a structure of a 5-membered to 7-membered cyclic substituent, except a case where $R^8$ and $R^9$ are simultaneously a hydrogen atom,

$R^{10}$ and $R^{11}$ may be taken together with a nitrogen atom carrying $R^{10}$ and $R^{11}$ to form a structure of a 5-membered to 7-membered cyclic substituent,
any one or more of $R^5$, $R^6$, $R^7$, $R^8$, $R^9$, $R^{10}$, $R^{11}$, and $R^{12}$ have a saturated or unsaturated 5 or more-membered cyclic substituent having heteroatoms, and
n is an integer of 1 to 100.

The photopolymerization initiator (A) can be used in combination with a photocationic polymerization initiator, a photoanionic polymerization initiator, or a thermal polymerization initiator, and can also be used for hybrid or dual polymerization, curing, and the like. Photopolymerization and thermal polymerization can be performed simultaneously or in any order. Photopolymerization is fast while unreacted monomers and oligomers may remain, so it is preferable to complete the remaining polymerization reactions and crosslinking reactions by thermal polymerization after photopolymerization. In addition, by using photoradical polymerization initiators of different types and different structures in combination and performing stepwise irradiation with light of different wavelengths, the curable product can be completely cured.

Examples of the light applied to the photopolymerization initiator (A) include active energy rays such as visible light, electron rays, ultraviolet rays, infrared rays, X-rays, $\alpha$-rays, $\beta$-rays, and $\gamma$-rays. Among them, it is preferable to use ultraviolet rays from the viewpoint of a balance among an active energy ray generator, a photopolymerization initiation rate, and safety. Examples of the light source of ultraviolet rays include a xenon lamp, a low-pressure mercury lamp, a high-pressure mercury lamp, a metal halide lamp, a UV-LED lamp, and a microwave-type excimer lamp, and from the viewpoint of high conversion efficiency of energy into light, easy increase in output, and no use of harmful mercury, a UV-LED lamp is more preferable.

In the photopolymerization initiator (A), the light irradiation energy required for generating radical active species is preferably in a range of 5 to 50,000 mJ/cm$^2$, more preferably 10 to 20,000 mJ/cm$^2$ in terms of irradiation energy (cumulative amount of light). When the irradiation energy is within this range, radicals having sufficient activity can be generated from (A), which is preferable.

The content of the photopolymerization initiator (A) in the curable composition varies depending on the type, content, and the like of monomers and oligomers in the curable composition. When the content is 0.1% by mass or more with respect to the entire curable composition, photopolymerization can be started immediately and the curable composition can be sufficiently cured at high speed, which is preferable. In addition, when (A) has an ethylenically unsaturated group, even if the curable composition contains 100% by mass of (A), it can be cured quickly and sufficiently like a normal curable composition. In addition, in order to suitably adjust the physical properties of the obtained cured product, (A) is preferably used in combination with another monomer or oligomer. In this case, the content of (A) is preferably 0.5 to 70% by mass, more preferably 1 to 50% by mass, and most preferably 2 to 30% by mass with respect to the entire curable composition.

The monomer and oligomer to be used in combination with (A) can be classified as a monofunctional monomer and a polyfunctional monomer or oligomer. The content of the monomers or oligomers to be used in combination is 0 to 99.9% by mass with respect to the entire curable composition, and is preferably 10 to 99.5% by mass, more preferably 30 to 99% by mass, and most preferably 50 to 90% by mass from the viewpoint of suitably adjusting the physical properties of the cured product.

Examples of the monofunctional monomer include compounds containing a (meth)acrylate group, a (meth) acrylamide group, a vinyl group, an allyl group, a styryl group, an acetylene group, and the like. These can be used alone or in combination of two or more. The content of the monofunctional monomer is preferably 0 to 90% by mass, more preferably 5 to 70% by mass, and most preferably 10 to 50% by mass with respect to the entire curable composition. The monofunctional monomer usually has a low viscosity, and by appropriately containing the monofunctional monomer, effects such as lowering the viscosity of the curable composition and improving workability can be expected.

Specific examples of the monofunctional monomer containing a (meth)acrylate group (excluding the photopolymerization initiator) include (meth)acrylates into which a linear alkyl group or hydroxyalkyl group having 1 to 18 carbon atoms or a branched or cyclic alkyl group or hydroxyalkyl group having 3 to 18 carbon atoms, alkyl carboxylic acid, alkyl sulfonic acid, or alkyl phosphoric acid is introduced; phenoxyalkylene glycol (meth)acrylates into which a functional group including a phenoxy group and an alkylene glycol group having 1 to 4 carbon atoms is introduced; (meth)acrylates containing an amino group such as dialkylaminoethyl (meth)acrylate and dialkylaminopropyl (meth)acrylamide into which an alkyl group having 1 to 6 carbon atoms is introduced; (meth)acrylates into which a cyclic structure is introduced such as benzyl (meth)acrylate, dicyclopentanyl (meth)acrylate, dicyclopentenyl (meth)acrylate, isobornyl (meth)acrylate, tetrahydrofurfuryl (meth)acrylate, and 2-methyl-2-adamantyl (meth)acrylate; and (meth)acrylates into which an epoxy group is introduced such as glycidyl (meth)acrylate and 4-hydroxybutyl (meth)acrylate glycidyl ether.

Specific examples of the monofunctional monomer containing a (meth)acrylamide group (excluding the photopolymerization initiator) include (meth)acrylamide, mono- or di-substituted (meth)acrylamide, (meth)acryloylmorpholine, and diacetone (meth)acrylamide. Examples of the mono- or di-substituted (meth)acrylamide include N-alkyl (meth)acrylamide and N,N-dialkyl (meth)acrylamide into which a linear alkyl group having 1 to 18 carbon atoms, a branched alkyl group having 3 to 18 carbon atoms, or a cyclic alkyl group having 3 to 18 carbon atoms is introduced; N-hydroxyalkyl (meth) acrylamide in which a hydroxyalkyl group having 1 to 6 carbon atoms is introduced; and N,N-dialkylaminopropyl (meth) acrylamide into which an alkyl group having 1 to 6 carbon atoms is introduced.

Specific examples of the monofunctional monomer containing a vinyl group, an allyl group, or a styryl group (excluding the photopolymerization initiator) include carboxylic acid vinyl esters or carboxylic acid allyl esters into which a linear carboxylic acid having 1 to 18 carbon atoms or a branched or cyclic carboxylic acid having 3 to 18 carbon atoms is introduced; alkyl vinyl ethers or alkyl allyl ethers into which a linear alkyl group having 1 to 18 carbon atoms or a branched or cyclic alkyl group having 3 to 18 carbon atoms is introduced; vinyl chloride; N-vinylpyrrolidone; N-vinylcaprolactam; N-vinyloxazoline; maleic acid; maleic anhydride; fumaric acid; itaconic acid; itaconic anhydride; unsaturated dicarboxylic acids mono- or diesterified with a linear alkyl group having 1 to 18 carbon atoms or a branched or cyclic alkyl group having 3 to 18 carbon atoms; vinyl carboxylic acid; vinyl sulfonic acid; vinyl phosphoric acid; allylamine; diallylamine; styrene; α-methylstyrene; α-methylstyrene dimer; and p-styrenesulfonic acid.

Examples of the polyfunctional monomer or oligomer include compounds containing two or more unsaturated groups such as a (meth)acrylate group, a (meth)acrylamide group, a vinyl group, an allyl group, a styrene group, and an acetylene group. These compounds may contain one kind of unsaturated group alone, or contain two or more kinds of unsaturated groups in combination. **In** addition, in order to obtain good curability, it is more preferable to use one or more (meth)acrylate groups or (meth)acrylamide groups as the unsaturated group. The content of the polyfunctional monomer (excluding the photopolymerization initiator) is preferably 0 to 95% by mass, more preferably 1 to 70% by mass, and most preferably 5 to 50% by mass with respect to the entire curable composition. By appropriately adjusting the content of the polyfunctional monomer, high strength and hardness, and excellent durability of the cured product obtained can be expected.

Examples of the polyfunctional monomer or oligomer include allyl (meth)acrylate, allyl (meth)acrylamide, diallylamine, alkyldiallylamine into which an alkyl group having 1 to 18 carbon atoms is introduced, alkylene glycol di(meth)acrylates, polyalkylene glycol di(meth)acrylates, bisphenol A diglycidyl ether acrylic acid adducts, alkoxylated bisphenol A diacrylates, polyester di(meth)acrylates, polycarbonate di(meth)acrylates, polyurethane di(meth)acrylates, and polyurethane di(meth)acrylamides. **In** addition, examples of the trifunctional or more polyfunctional monomer include pentaerythritol tri(meth)acrylate, pentaerythritol tetra(meth)acrylate, trimethylolpropane tri(meth)acrylate, dipentaerythritol tri(meth)acrylate, ditrimethylolpropane tetra(meth)acrylate, dipentaerythritol tetra(meth)acrylate, dipentaerythritol penta(meth)acrylate, dipentaerythritol hexa(meth)acrylate, tri(meth)acryloyloxyethoxytrimethylolpropane, glycerin polyglycidyl ether poly(meth)acrylate, isocyanuric acid ethylene oxide-modified tri(meth)acrylate, ethylene oxide-modified dipentaerythritol penta(meth)acrylate, ethylene oxide-modified dipentaerythritol hexa(meth)acrylate, ethylene oxide-modified pentaerythritol tri(meth)acrylate, ethylene oxide-modified pentaerythritol tetra(meth)acrylate, and succinate-modified pentaerythritol tri(meth)acrylate. These polyfunctional monomers or oligomers can be used alone or in combination of two or more.

The photopolymerization initiator (A) of the present disclosure has high photoinitiating properties without using a sensitizer. Furthermore, by using a general-purpose sensitizer in combination, further improvement in polymerization initiation property and physical properties of the cured product after curing can be expected. The sensitizer that can be used in combination with (A) is not particularly limited. Examples thereof include benzophenones; unsaturated ketones typified by anthracene derivatives and the like; 1,2-diketone derivatives typified by benzyl, camphorquinone and the like; benzoin derivatives; anthraquinone derivatives; thioxanthone derivatives; coumarin derivatives; tertiary amines; thiols; and disulfides. These can be used in any ratio as necessary, and can be used alone or in combination of two or more.

Specific examples of the sensitizer that can be used in combination include anthracene-based sensitizers such as 9,10-dibutoxy anthracene, 9,10-diethoxy anthracene, 9,10-dipropoxy anthracene, and 9,10-bis(2-ethylhexyloxy) anthracene and thioxanthone-based sensitizers such as 2,4-diethyl thioxanthone, 2-isopropyl thioxanthone, and 4-isopropyl thioxanthone. Typical examples of commercially available products include DBA and DEA (manufactured by Kawasaki Kasei Chemicals Ltd.) as anthracene-based sensitizers and DETX and ITX (manufactured by Lambson Ltd.) as thioxanthone-based sensitizers. The content of the sensitizer is not particularly limited, and is preferably 0.5 to 5.0% by mass, and more preferably 0.8 to 3.0% by mass with respect to the entire curable composition. When the content of the sensitizer is within this range, the curability of the curable composition is improved, and the obtained cured product has good durability and yellowing resistance.

Examples of other polymerization initiators that can be used in combination with the photopolymerization initiator (A) of the present disclosure include benzoins such as benzoin and benzoin alkyl ethers; acetophenones such as acetophenone and 2-hydroxy-2-methyl-1-phenylpropan-1-one; anthraquinones; thioxanthones; ketals; benzophenones; aminobenzophenones; aminoacetophenones; and xanthones. These can be used in any ratio as necessary, and can be used alone or in combination of two or more.

The active energy ray curable composition can be used without containing an organic solvent. **In** addition, in order to improve workability such as coating properties, an organic solvent can be added as necessary to control the

viscosity of the liquid. The added organic solvent may be removed in advance before photo-curing. The composition also can be cured while containing the organic solvent. Furthermore, the organic solvent can be removed after curing. The method for removing the organic solvent can be appropriately selected according to the method and purpose of the curable composition and the obtained cured product. The amount of the organic solvent to be added is not particularly limited, and is preferably 80% by mass or less and more preferably 50% by mass or less with respect to the entire active energy ray curable composition from the viewpoint of reducing energy and time required for removing the organic solvent.

The organic solvent can be used for the curable composition. Examples of the organic solvent that can be used include alcohols such as methanol and isopropanol; ketones such as acetone, methyl ethyl ketone, and cyclohexanone; esters such as ethyl acetate, propyl acetate, methyl lactate, and ethyl lactate; alkylene glycols such as ethylene glycol and propylene glycol; polyalkylene glycols such as polyethylene glycol and polypropylene glycol; glycol ethers such as ethoxydiethylene glycol and methoxypropylene glycol; glycol esters such as propylene glycol acetate; ethers such as tetrahydrofuran, methyltetrahydrofuran, cyclopentyl methyl ether, methyltetrahydropyran, and methyl tert-butyl ether toluene; aromatic hydrocarbons such as xylene; aliphatic hydrocarbons such as hexane and cyclohexane; amides such as N,N'-dimethylformamide and dimethylacetamide; amide ethers such as β-methoxy-N,N-dimethylpropionamide and β-butoxy-N,N-dimethylpropionamide; pyrrolidones such as 2-pyrrolidone and N-methylpyrrolidone; piperidines such as N-methylpiperidine; halogenated hydrocarbons such as methylene chloride, chloroform, and dichloroethane; sulfoxides such as dimethylsulfoxide; and imidazolidinones such as 1,3-dimethyl-2-imidazolidinone. These organic solvents can be used alone or in combination of two or more.

The photopolymerization initiator of the present disclosure can be suitably used for active energy ray curable inks such as an active energy ray curable flexographic ink, an active energy ray curable offset ink, an active energy ray curable screen ink, and an active energy ray curable inkjet ink; an active energy ray curable nail cosmetic composition used for gel nails, and the like; an active energy ray curable pressure-sensitive adhesive composition; an active energy ray curable adhesive composition; an active energy ray curable sealant composition used for sealing materials or sealants; an active energy ray curable coating composition used for paints and coating agents for automobiles, electrical appliances, furniture, and the like; an active energy ray curable decorative sheet composition used for decorative sheets used for surface coating of automobiles and electrical appliances, and the like; a coating composition with self-repairing properties; an active energy ray curable self-repairing material composition used for three-dimensional modeling objects, nail decoration materials, dental materials, automobile exterior protection, functional members such as decorative films, devices, and the like; an active energy ray curable elastomer composition used for elastomer materials used for transparent pressure-sensitive adhesive sheets, cushioning materials, packing, vibration-proofing materials, sound-absorbing materials, printing plates, sealing materials, abrasives, and the like; an active energy ray curable three-dimensional modeling ink used for model materials and support materials for 3D printers or for threedimensionally decorating a film in a three-dimensional shape having irregularities; an active energy ray curable dental material composition; an active energy ray curable type photosensitive composition; an active energy ray curable hydrogel composition; an active energy ray curable intraocular implant material composition; and the like. In addition, the obtained hydrogel composition can also be suitably used as materials in a wide variety of fields such as hygiene fields such as superabsorbent resins, disposable diapers, and soft contact lenses; various coating fields such as ship bottom paints, antifogging materials, and antifouling paints; medical fields such as medical device surface coatings and artificial organs; civil engineering and construction fields such as soil improvers; agricultural fields such as water retention materials; and shock absorbing materials.

EXAMPLES

Hereinbelow, the present invention is described still more specifically by showing Examples and Comparative Examples; however, the present invention is not limited to these Examples. In the following, all descriptions of "parts" and "%" are on a mass basis unless otherwise specified.

(1-1) Infrared absorption spectrum analysis (IR analysis)

IR analysis was performed with the following apparatus.
Nicolet iS50 (manufactured by Thermo Fisher Scientific K.K.)

(1-2) Liquid chromatography mass spectroscopy (LC-MS analysis)

LC-MS analysis was performed with the following apparatus under the following conditions.

Column: XBridge C18, 6 mm × 150 mm, 3.5 μm (manufactured by Nihon Waters K.K.)
Eluent conditions: water/methanol/1%-formic acid aqueous solution = 60/30/10

Measuring wavelength: 258 nm; column oven: 40°C

(1-3) Nuclear magnetic resonance spectrum analysis (NMR analysis)

$^1$H-NMR analysis was performed with an apparatus of 400 MHz manufactured by JEOL Ltd.

(1-4) Gas chromatography analysis (GC analysis)

The conditions of GC analysis were performed with the following apparatus.

Apparatus: GC-2025 (manufactured by Shimadzu Corporation)
Column: DB-1 (manufactured by Agilent Technologies Japan, Ltd.)

(1-5) Gel permeation chromatography analysis (GPC analysis)

GPC analysis was performed with the following apparatus under the following conditions.

Apparatus: Prominence-I LC-2030C (manufactured by Shimadzu Corporation)
Guard column: one guard column of KF-G of Shodex (manufactured by Showa Denko K.K.)
Column: one column of KF-803 of Shodex (manufactured by Showa Denko K.K.) Column temperature: 40°C; mobile phase: tetrahydrofuran (THF)
Speed of liquid feeding: 0.5 mL/min; standard sample: polystyrene

Benzophenone-based compounds (a1), heterocyclic compounds (a2), and compounds (a5) capable of reacting with (a1) or (a2) used for Examples are shown below.

(2-1) Benzophenone-based compound (a1)

a1-1: Methyl 4-benzoylbenzoate
a1-2: 3,4-Benzophenone dicarboxylic anhydride
a1-3: 2-(4-Biphenylylcarbonyl)benzoic acid
a1-4: 4-(4-Carboxybenzoyl)2,3-diethylbenzoic acid
a1-5: 4-(4-Methoxybenzoyl)benzoic acid
a1-6: 3,3',4,4'-Benzophenone tetracarboxylic dianhydride

(2-2) Heterocyclic compound (a2)

a2-1: Piperazine
a2-2: Morpholine
a2-3: Hydroxyethyl maleimide
a2-4: Tetrahydrofurfuryl alcohol
a2-5: Tetrahydrofuran-2-acetic acid chloride
a2-6: Tetrahydrofurfurylamine
a2-7: 2-(Hydroxymethyl)-15-crown 5-ether
a2-8: 1-Piperidine ethanol
a2-9: 4-(2-Hydroxyethyl)morpholine
a2-10: Tetrahydro-2H-thiopyran-4-ol

(2-3) Compound (a5) capable of reacting with (a1) or (a2)

a5-1: Acrylic acid chloride
a5-2: 4-Hydroxybutyl acrylate glycidyl ether
a5-3: Glycidyl methacrylate
a5-4: Ethylene glycol
a5-5: Butylene oxide
a5-6: Methanol
a5-7: Octadecyl glycidyl ether
a5-8: Ion-exchanged water

a5-9:1,2-Epoxyoctane
a5-10: 4-Hydroxybutyl acrylate
a5-11: Allyl alcohol
a5-12: N-(2-Hydroxyethyl)acrylamide

Polyols (B1), amine compounds (B2), monoalcohol compounds (B3), isocyanate compounds (C1), and carboxylic acid compounds (C2) used for Examples are shown below.

(2-4) Polyol (B1)

B1-1: UH-100 (a polycarbonate diol of 1,6-HD, manufactured by Ube Industries, Ltd., number average molecular weight: 1,000)
B1-2: KURARAY POLYOL P-1010 (a polyester polyol, manufactured by Kuraray Co., Ltd., number average molecular weight: 1,000)
B1-3: KF-6000 (reactive silicone oil, carbinol-modified (both-terminal type), manufactured by Shin-Etsu Chemical Co., Ltd., hydroxyl value: 120 mgKOH/g, number average molecular weight: 900)
B1-4: UNIOL D-1000 (polypropylene glycol, manufactured by NOF Corporation, number average molecular weight: 1,000)
B1-5: GI-2000 (polybutadienediol, manufactured by Nippon Soda Co., Ltd., number average molecular weight: 2,000)
B1-6: KURARAY POLYOL P-5010 (a polyester polyol, manufactured by Kuraray Co., Ltd., number average molecular weight: 5,000)
B1-7: PEG-300 (polyethylene glycol, number average molecular weight: 300)
B1-8: PTMG 650 (polytetramethylene ether glycol, number average molecular weight: 650)

(2-5) Amine compound (B2)
B2-1: D-400 (polyetheramine, manufactured by Mitsui Fine Chemicals, Inc., number average molecular weight: 400)
(2-6) Monoalcohol compound (B3)

B3-1: Hydroxyethyl acrylate
B3-2: N-(2-Hydroxyethyl)acrylamide (registered trademarks "Kohshylmer" and "HEAA", manufactured by KJ Chemicals Corporation)
B3-3: 4-Hydroxybutyl acrylate
B3-4: 3-Hydroxypropyl methacrylamide
B3-5: Hydroxyethyl maleimide
B3-6: Methanol
B3-7: 4-Hydroxybutyl vinyl ether
B3-8: Ethylene glycol monoallyl ether
B3-9: (6-Hydroxyhexyl)methacrylamide

(2-7) Isocyanate compound (C1)

C1-1: 2-Acryloyloxyethyl isocyanate
C1-2: Isophorone diisocyanate
C1-3: Toluene diisocyanate
C1-4: 1,5-Pentamethylene diisocyanate
C1-5: 1,3-bis(isocyanatomethyl)cyclohexane
C1-6: Trimer of isophorone diisocyanate (nurate) (VESTANAT T1890/100, manufactured by Evonik Industries AG)

(2-8) Carboxylic acid compound (C2)

C2-1: Adipic acid
C2-2: Sebacic acid

Photopolymerization initiators (D), monofunctional monomers (E), polyfunctional monomers or oligomers (F), and other components (G) used for Examples and Comparative Examples are shown below.

(3-1) Photopolymerization initiator (D)

D-1: 1-Hydroxycyclohexyl phenyl ketone
D-2: Benzophenone
D-3: Isopropylthioxanthone
D-4: Diphenyl(2,4,6-trimethylbenzoyl)phosphine oxide
D-5: Escacure KIP-150 (manufactured by IGM Rseins B.V)
D-6: Camphorquinone

(3-2) Monofunctional Monomer (E)

E-1: Diethylacrylamide (registered trademarks "Kohshylmer" and "DEAA", manufactured by KJ Chemicals Corporation)
E-2: Isobornyl acrylate
E-3: Acryloylmorpholine (registered trademarks "Kohshylmer" and "ACMO", manufactured by KJ Chemicals Corporation)
E-4: Tetrahydrofurfuryl acrylate
E-5: 4-t-Butylcyclohexyl acrylate (registered trademark "Kohshylmer", manufactured by KJ Chemicals Corporation)
E-6: Phenoxyethyl acrylate
E-7: 4-Hydroxybutyl acrylate
E-8: Dimethylacrylamide (registered trademarks "Kohshylmer" and "DMAA", manufactured by KJ Chemicals Corporation)
E-9: N-Vinylpyrrolidone
E-10: N-Octylacrylamide (registered trademark "Kohshylmer", manufactured by KJ Chemicals Corporation)
E-11: N-(2-Hydroxyethyl)acrylamide (registered trademarks "Kohshylmer" and "HEAA", manufactured by KJ Chemicals Corporation)
E-12: n-Dodecyl acrylate
E-13: Isobornyl methacrylate
E-14: Hydroxyethyl methacrylate
E-15: 2-Methacryloyloxyethyl acid phosphate

(3-3) Polyfunctional monomer or oligomer (F)

F-1: A-400 (polyethylene glycol No. 400 diacrylate, manufactured by Shin-Nakamura Chemical Co., Ltd.)
F-2: EBECRYL 8807 (an aliphatic bifunctional urethane acrylate, manufactured by Daicel-Allnex Ltd., average molecular weight: 1000)
F-3: Urethane diacrylate (SHIKOH UV6630, manufactured by Mitsubishi Chemical Corporation)
F-4: Polyethylene glycol (20)-introduced bisphenol A diacryl (NK Ester A-BPE-20, manufactured by Shin-Nakamura Chemical Co., Ltd.)
F-5: Hexanediol diacrylate
F-6: Polyether-based urethane acrylamide (registered trademark "Quick Cure", manufactured by KJ Chemicals Corporation)
F-7: Dipentaerythritol hexaacrylate (
F-8: Urethane diacrylate (SHIKOH UV3000, manufactured by Mitsubishi Chemical Corporation)
F-9: Dimethylol-tricyclodecane diacrylate
F-10: Polyethylene glycol (10)-introduced bisphenol A diacryl (NK Ester A-BPE-10, manufactured by Shin-Nakamura Chemical Co., Ltd.)
F-11: Pentaerythritol triacrylate
F-12: Polyethylene glycol (10) dimethacrylate
F-13: Ethylene bisacrylamide
F-14: Polyester-based urethane acrylamide (registered trademark "Quik Cure", manufactured by KJ Chemicals Corporation)
F-15: Trimethylolpropane triacrylate
F-16: Polycarbonate-based urethane acrylamide (registered trademark "Quick Cure", manufactured by KJ Chemicals Corporation)
F-17: Bisphenol A epoxy acrylate oligomer (Miramer PE-210, manufactured by Miwon Specialty Chemical Co., Ltd.)

(3-4) Other components (G)

G-1: TEGO Rad 2100 (silicon acrylate having polydimethylsiloxane structure, manufactured by Evonik Industries AG)
G-2: Pentaerythritol tetrakis (3-mercaptobutyrate)
G-3: MEK-ST-40 (colloidal silica dispersion, manufactured by Nissan Chemical Corporation)
G-4: Omnipol ASA (manufactured by IGM Resins B.V)
G-5: Carbon black dispersion (manufactured by Mitsubishi Chemical Corporation)/BYK-JET 9151 (pigment dispersant, maleimide-styrene copolymer having ammonium salt structure, manufactured by BYK-Chemie GmbH) = 1/2 (weight ratio) mixed liquid
G-6: VALIFAST BLUE 1613 (manufactured by Orient Chemical Industries Co., Ltd.)
G-7: Petrotac 100V (manufactured by Tosoh Corporation)
G-8: Reolosil QS-30 (manufactured by Tokuyama Corporation)
G-9: Methacrylic acid/methyl methacrylate/styrene copolymer binder
G-10: Azobisisobutyronitrile

(3-5) Organic solvent (H)

H-1: Ethyl acetate
H-2: 3-Methoxy-N,N-dimethylpropanamide (registered trademarks "Kohshylvent" and "KJCMPA", manufactured by KJ Chemicals Corporation)
H-3: Methyl ethyl ketone
H-4: Dimethylformamide
H-5: N-Methylpyrrolidone

Example 1: Synthesis of photopolymerization initiator (A-1)

To a 500 mL capacity flask equipped with a reflux condenser tube, a stirrer, a thermometer, and a dropping funnel, 35.9 g of piperazine (a2-1) and 50 g of N,N-dimethylformamide (DMF) were added, and 37.7 g of acrylic acid chloride (a5-1) was added dropwise while stirring at 0°C and reacted for 1 hour to obtain 1-acryloylpiperazine. Thereafter, 100 g of methyl 4-benzoylbenzoate (a1-1) and 3.1 g of triethylamine (TEA) were added, and the mixture was reacted at 65°C for 2 hours while stirring. After the reaction was completed, the reaction solution was purified to obtain a target pale yellow viscous liquid (purity: 95%). The obtained viscous liquid was subjected to IR analysis, and the C=O specific absorption (1650 $cm^{-1}$) of an amide group was detected, and the C=O specific absorption (1725 $cm^{-1}$) of an ester group was not detected, so that generation of an amide group and disappearance of an ester group could be confirmed. By $^1$H-NMR analysis, 8.60 to 7.90 ppm (9H, aromatic ring); 6.34 ppm (H, -CH= of acrylamide group); 6.22 ppm and 5.74 ppm (2H, =CH2 of acrylamide group); and 3.80 ppm and 3.60 ppm (4H, -N-CH2- of piperazine ring) were detected, and the presence of a benzophenone group, an acryloyl group, and a piperazine group could be confirmed. In addition, the molecular weight shown from the molecular ion peak of the mass spectrum of LC-MS was 348, which was consistent with the molecular weight of the target compound (Table 2), and generation of a photopolymerization initiator (A-1) having the structure shown in Table 2 was confirmed.

Example 2: Synthesis of photopolymerization initiator (A-2)

Using a similar apparatus, 100 g of (a1-2), 34.5 g of (a2-2), 1.5 g of TEA, and 50 g of DMF were added, and the mixture was reacted at 65°C for 2 hours, then 79.3 g of (a5-2) and 0.4 g of triphenylphosphine (TPP) were added, and the mixture was reacted at 70°C for 3 hours. After the reaction was completed, the reaction solution was purified to obtain a pale yellow viscous liquid (purity: 97%). Similarly, generation of an ester group and an amide group (1725 $cm^{-1}$ of C=O of an ester group and 1650 $cm^{-1}$ of C=O of an amide group were detected) and disappearance of an acid anhydride group (no C=O specific absorption at 1770 $cm^{-1}$ and 1851 $cm^{-1}$ of an acid anhydride group was detected) were confirmed by IR analysis. By $^1$H-NMR analysis, 8.60 to 7.95 ppm (8H, aromatic ring); 6.56 ppm (H, -CH= of acrylate group); 4.28 ppm and 4.32 ppm (2H, =CH2 of acrylate group); 3.63 ppm (8H, -CH2-O- in morpholine group); and 3.41 ppm (8H, -N-CH2- in morpholine group) were detected, and the molecular weight (540) obtained by LC-MS analysis was consistent with the molecular weight of the target compound (Table 2). Based on these results, the generation of a photopolymerization initiator (A-2) having the structure shown in Table 2 was confirmed.

Example 3: Synthesis of photopolymerization initiator (A-3)

Using a similar apparatus, 100 g of (a1-3), 47.2 g of (a2-3), and 39.4 g of thionyl chloride were stirred at 0°C for 10 minutes and then heated to 70°C and reacted for 3 hours. After the reaction was completed, the reaction solution was purified to obtain a pale yellow viscous liquid (purity: 93%). Similarly, by IR analysis of the obtained viscous liquid, generation of an ester group (1725 $cm^{-1}$) was confirmed, and by $^1H$-NMR analysis, 8.50 to 7.80 ppm (13H, aromatic ring); 7.05 ppm (2H, -HC=CH- of maleimide group); 4.65 ppm (2H, -O-CH2-); and 3.78 ppm (2H, -N-CH2-) were detected, and the molecular weight (425) obtained by LC-MS analysis was consistent with the molecular weight of the target compound (Table 2). Based on these results, the generation of a photopolymerization initiator (A-3) having the structure shown in Table 2 was confirmed.

Example 4: Synthesis of photopolymerization initiator (A-4)

(a1-3) was changed to (a1-4), and a reaction was performed in the same manner as in Example 3 to obtain a pale yellow viscous liquid (purity: 97%). From the results of IR analysis, $^1H$-NMR analysis (described below), and LC-MS analysis (molecular weight: 495) of the obtained viscous liquid, generation of a photopolymerization initiator (A-4) having the structure shown in Table 2 was confirmed. $^1H$-NMR: 8.62 to 7.97 ppm (6H, aromatic ring); 4.52 ppm (4H, -COO-CH2-); 4.02 ppm (2H, -CH-O- in two tetrahydrofuran groups); 3.82 ppm (4H, -CH2-O- in two tetrahydrofuran groups); 1.63 to 2.02 ppm (8H, -CH2-CH2- in two tetrahydrofuran groups).

Example 5: Synthesis of photopolymerization initiator (A-5)

Using a similar apparatus, 100 g of (a1-5), 55.5 g of (a5-3), and 50 g of DMF were mixed, and reacted at 65°C for 2 hours. Then the reaction solution was cooled to 0°C, 58.0 g of (a2-5) was added thereto, and the mixture was stirred for 10 minutes, then heated to 70°C, and reacted for 3 hours. After the reaction was completed, the reaction solution was purified to obtain a pale yellow viscous liquid (purity: 87%). IR analysis of the obtained viscous liquid, generation of an ester group (1725 $cm^{-1}$) was confirmed. By $^1H$-NMR analysis, the presence of a benzophenone group (8.50 to 7.85 ppm, 6H, aromatic ring), a tetrahydrofuran group (the same as in Example 4), and a methacrylate group (5.97 ppm and 5.48 ppm, 2H, =CH2; 1.96 ppm, 3H, -CH3) was confirmed. The results of LC-MS analysis showed that the molecular weight of the target compound was 511 (Table 2). Based on these results, the generation of a photopolymerization initiator (A-5) having the structure shown in Table 2 was confirmed.

Example 6: Synthesis of photopolymerization initiator (A-6)

Using a similar apparatus, 100 g of (a1-6), 63.4 g of (a2-4), 3.1 g of TEA, and 50 g of DMF were mixed, and reacted at 65°C for 2 hours. The reaction solution was cooled to 0°C, 38.5 g of (a5-4) and 73.8 g of thionyl chloride were added thereto, and the mixture was stirred for 10 minutes, then reacted at 70°C for 3 hours, and purified to obtain a pale yellow viscous liquid (purity: 91%). By IR analysis of the obtained viscous liquid, generation of an ester group (1725 $cm^{-1}$) and disappearance of an acid anhydride group were confirmed. By $^1H$-NMR analysis, the presence of a benzophenone group (8.62 to 7.95 ppm, 6H, aromatic ring) and a tetrahydrofuran group (the same as in Example 4) was confirmed. The LC-MS analysis value molecular weight (615) was consistent with the molecular weight of the target compound (Table 2). Based on these results, the generation of a photopolymerization initiator (A-6) having the structure shown in Table 2 was confirmed.

Example 7: Synthesis of photopolymerization initiator (A-7)

Using a similar apparatus, 100 g of (a1-6), 54.1 g of (a2-2), 3.1 g of TEA, and 50 g of DMF were mixed, and reacted at 65°C for 2 hours. The reaction solution was cooled to 0°C, 44.8 g of (a5-5) and 0.8 g of TPP were added thereto, and the mixture was stirred for 10 minutes, then reacted at 70°C for 3 hours, and purified to obtain a pale yellow viscous liquid (purity: 84%). Similarly, by IR analysis of the obtained viscous liquid, the generation of an ester group and an amide group and disappearance of an acid anhydride group were confirmed. By $^1H$-NMR analysis, the presence of a benzophenone group and a morpholine group was confirmed. By LC-MS analysis, it was confirmed that the molecular weight (641) was consistent with that of the target compound (Table 2). From these results, the generation of a photopolymerization initiator (A-7) having the structure shown in Table 2 was confirmed.

Example 8: Synthesis of photopolymerization initiator (A-8)

Using a similar apparatus, 100 g of (a1-6), 63.7 g of (a2-4), and 50 g of DMF were mixed, and reacted at 70°C for 4

hours. The reaction solution was cooled to 0°C, 19.9 g of (a5-6) and 73.8 g of thionyl chloride were added thereto, and the mixture was stirred for 10 minutes, then reacted at 70°C for 3 hours. After the reaction was completed, the reaction solution was purified to obtain a pale yellow viscous liquid (purity: 96%). Similarly, by IR analysis of the obtained viscous liquid, generation of an ester group and disappearance of an acid anhydride group were confirmed. By $^1$H-NMR analysis, the presence of a benzophenone group and a tetrahydrofuran group was confirmed. By LC-MS analysis, it was confirmed that the molecular weight (555) was consistent with that of the target compound (Table 2). From these results, the generation of a photopolymerization initiator (A-8) having the structure shown in Table 2 was confirmed.

Example 9: Synthesis of photopolymerization initiator (A-9)

Using a similar apparatus, 100 g of (a1-6), 54.1 g of (a2-2), 3.1 g of TEA, and 50 g of DMF were mixed, and the mixture was reacted at 65°C for 2 hours, then 202.7 g of (a5-7) was added, and the mixture was reacted at 85°C for 5 hours. After completion of the reaction, the reaction solution was purified to obtain a pale yellow viscous liquid (purity: 81%). Similarly, by IR analysis of the obtained viscous liquid, the generation of an ester group and an amide group and disappearance of an acid anhydride group were confirmed. By $^1$H-NMR analysis, the presence of a benzophenone group and a morpholine group was confirmed. By LC-MS analysis, it was confirmed that the molecular weight (1150) was consistent with that of the target compound (Table 2). From these results, the generation of a photopolymerization initiator (A-9) having the structure shown in Table 2 was confirmed.

Example 10: Synthesis of photopolymerization initiator (A-10)

Using a similar apparatus, 100 g of (a1-6), 27.0 g of (a2-2), 1.5 g of TEA, and 50 g of DMF were mixed, and the mixture was reacted at 65°C for 2 hours, then 11.2 g of ion-exchanged water was added, and the mixture was reacted at 65°C for 3 hours. After completion of the reaction, the reaction solution was purified to obtain a pale yellow viscous liquid (purity: 97%). Similarly, by IR analysis of the obtained viscous liquid, the generation of an amide group and disappearance of an acid anhydride group were confirmed. By $^1$H-NMR analysis, the presence of a benzophenone group and a morpholine group was confirmed. By LC-MS analysis, it was confirmed that the molecular weight (427) was consistent with that of the target compound (Table 2). From these results, the generation of a photopolymerization initiator (A-10) having the structure shown in Table 2 was confirmed.

Examples 11, 14, and 15: Synthesis of photopolymerization initiators (A-11), (A-14), and (A-15)

The reactions of Examples 11, 14, and 15 were performed in the same manner as in Example 7 using the raw material compounds shown in Table 1 to obtain pale yellow viscous liquids. Similarly, by IR analysis, $^1$H-NMR analysis, and LC-MS analysis, generation of photopolymerization initiators (A-11), (A-14), and (A-15) having the structures shown in Table 2 was confirmed.

Examples 12, 17, 19, and 20: Synthesis of photopolymerization initiators (A-12), (A-17), (A-19), and (A-20)

The reactions of Examples 12, 17, 19, and 20 were performed in the same manner as in Example 6 using the raw material compounds shown in Table 1 to obtain pale yellow viscous liquids. Similarly, by IR analysis, $^1$H-NMR analysis, and LC-MS analysis, generation of photopolymerization initiators (A-12), (A-17), (A-19), and (A-20) having the structures shown in Table 2 was confirmed.

Example 13: Synthesis of photopolymerization initiator (A-13)

Using a similar apparatus, 100 g of (a1-7), 135.6 g of 2-(hydroxymethyl)-15 crown 5-ether, 1.5 g of TEA, and 50 g of DMF were mixed, and reacted at 65°C for 2 hours. After completion of the reaction, the reaction solution was purified to obtain a pale yellow viscous liquid (purity: 84%). Similarly, by IR analysis, $^1$H-NMR analysis (described below), and LC-MS analysis, generation of a photopolymerization initiator (A-13) having the structure shown in Table 2 was confirmed.

$^1$H-NMR: 8.61 to 7.78 ppm (6H, aromatic ring); 5.02 ppm (2H, -COO-CH-); 3.68 to 4.01 ppm (28H, -CH2-O- in crown ether).

Example 16: Synthesis of photopolymerization initiator (A-16)

Using a similar apparatus, 100 g of (a1-6), 88.2 g of (a5-3), 10.0 g of tetrabutylammonium bromide, and 50 g of DMF were mixed, and reacted at 70°C for 4 hours. The reaction solution was cooled to 0°C, 54.1 g of (a2-4) and 73.8 g of thionyl chloride were added thereto, and the mixture was stirred for 10 minutes, then reacted at 70°C for 3 hours. After

completion of the reaction, the reaction solution was purified to obtain a pale yellow viscous liquid (purity: 83%). Similarly, by IR analysis of the obtained viscous liquid, generation of an ester group and disappearance of an acid anhydride group were confirmed. By $^1$H-NMR analysis, the presence of a benzophenone group, a tetrahydrofuran group, and a methacrylate group was confirmed. By LC-MS analysis, it was confirmed that the molecular weight (811) was consistent with that of the target compound (Table 2). From these results, the generation of a photopolymerization initiator (A-16) having the structure shown in Table 2 was confirmed.

Example 18: Synthesis of photopolymerization initiator (A-18)

The reaction of Examples 18 was performed in the same manner as in Example 16 using the raw material compounds shown in Table 1 to obtain a pale yellow viscous liquid. Similarly, by IR analysis, $^1$H-NMR analysis (described below), and LC-MS analysis, generation of a photopolymerization initiator (A-18) having the structure shown in Table 2 was confirmed.

$^1$H-NMR: 4.91 ppm (2H, -O-CH-), 2.71 ppm (4H, -CH2-N-), 2.22 ppm (8H, -N-CH2-in thiopyran group); 1.53 to 1.41 ppm (10H, -CH2-CH2- in thiopyran group).

[Table 1]

| | | Photopolymerization initiator (A) | Compound (al) (a3) | (g) | Compound (a2) (a4) | (g) | Compound (a5) | (g) |
|---|---|---|---|---|---|---|---|---|
| | 4 | A-4 | al-4 | 100.0 | a2-4 | 62.9 | - | - |
| | 11 | A-11 | al-6 | 100.0 | a2-6 | 62.8 | - | - |
| | | | al-6 + a2-6 | 162.8 | - | - | a5-5 | 44.8 |
| | 12 | A-12 | al-6 | 100.0 | a2-2 | 54.1 | - | - |
| | | | al-6 + a2-2 | 154.1 | a2-2 | 54.1 | - | - |
| | 14 | A-14 | al-6 | 100.0 | a2-8 | 80.2 | - | - |
| | | | al-6 + a2-8 | 180.2 | - | - | a5-7 | 202.7 |
| | 15 | A-15 | al-6 | 100.0 | a2-9 | 81.4 | - | - |
| | | | al-6 + a2-9 | 181.4 | - | - | a5-9 | 79.6 |
| | 17 | A-17 | al-6 | 100.0 | a2-2 | 54.1 | - | - |
| | | | al-6 + a2-2 | 154.1 | - | - | a5-10 | 89.5 |
| | 18 | A-18 | al-6 | 100.0 | - | - | a5-7 | 202.7 |
| | | | a1-6 + a5-7 | 302.7 | a2-10 | 73.4 | - | - |
| | 19 | A-19 | al-6 | 100.0 | a2-2 | 54.1 | - | - |
| | | | al-6 + a2-2 | 154.1 | - | - | a5-11 | 36.0 |
| | 20 | A-20 | al-6 | 100.0 | a2-9 | 81.4 | - | - |
| | | | a1-6 + a2-9 | 181.4 | - | - | a5-12 | 71.5 |

[Table 2-1]

| | | Photopolymerization initiator (A) | Molecular weight | 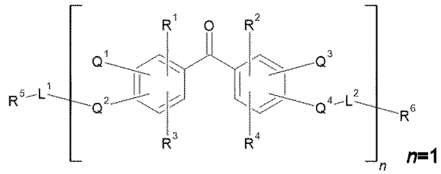 *n*=1 | | | | | | | | | | | | | | |
|---|---|---|---|---|---|---|---|---|---|---|---|---|---|---|---|---|---|---|
| | | | | $R^1$ to $R^4$ | | | $Q^1$ | | $Q^2$ | | $L^1$ | $R^5$ | $Q^3$ | | $Q^4$ | | $L^2$ | $R^6$ |
| Examples | 1 | A-1 | 474 | - | - | - | 4 | 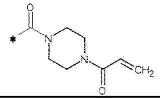 | - | - | | | - | - | - | - | | |
| | 2 | A-2 | 540 | - | - | - | 3 | 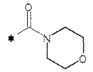 | 4 | 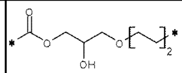 | 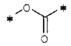 | 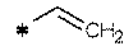 | - | - | - | - | | |
| | 3 | A-3 | 425 | $R^1$ | 4' | 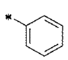 | 2 | 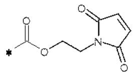 | - | - | | | - | - | - | - | | |
| | 4 | A-4 | 495 | $R^1$<br>$R^3$ | 2<br>3 | 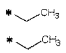 | 4 | 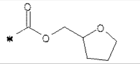 | - | - | | | 4' | 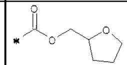 | - | - | | |
| | 5 | A-5 | 511 | $R^1$ | 4' | 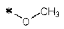 | 4 | 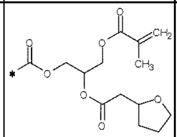 | - | - | | | - | - | - | - | | |
| | 6 | A-6 | 615 | - | - | - | 3 | 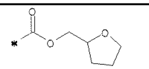 | 4 | 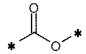 | | 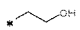 | 3' | 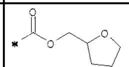 | 4' | 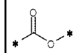 | | 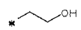 |
| | 7 | A-7 | 641 | - | - | - | 3 | 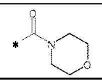 | 4 | 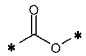 | | 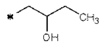 | 3' | 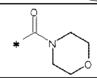 | 4' | 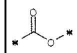 | | 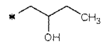 |
| | 8 | A-8 | 555 | - | - | - | 3 | 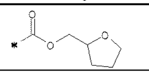 | 4 | 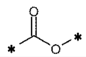 | | | 3' | 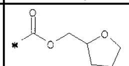 | 4' | 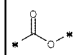 | | |
| | 9 | A-9 | 1150 | - | - | - | 3 | 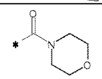 | 4 | 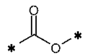 | | 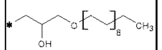 | 3' | 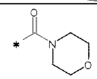 | 4' | 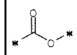 | | 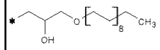 |
| | 10 | A-10 | 427 | - | - | - | 3 | 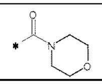 | 4 | 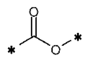 | | | 3' | 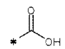 | 4' | 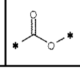 | | |

* is a connecting site.

$R^1$ to $R^4$, $Q^2$-$L^1$-$R^5$, $Q^4$-$L^2$-$R^6$, $Q^3$: - represents a hydrogen atom.

$L^1$, $L^2$: In the absence of description, a direct bond is represented.

[Table 2-2]

| | | Photopolymerization initiator (A) | Molecular weight | 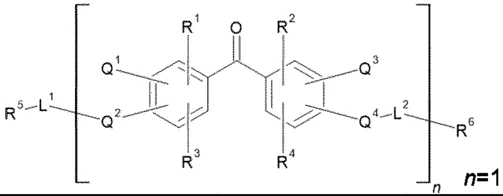 $n$=1 | | | | | | | | | | | | | | | |
|---|---|---|---|---|---|---|---|---|---|---|---|---|---|---|---|---|---|---|---|
| | | | | $R^1$ to $R^4$ | | | $Q^1$ | | $Q^2$ | | $L^1$ | $R^5$ | $Q^3$ | | $Q^4$ | | $L^2$ | $R^6$ |
| Examples | 11 | A-11 | 669 | - | - | - | 3 | 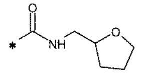 | 4 | 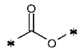 | | 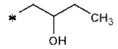 | 3' | | 4' | | | |
| | 12 | A-12 | 635 | - | - | - | 3 | 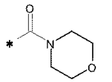 | 4 | 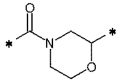 | | 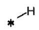 | 3' | | 4' | | | |
| | 13 | A-13 | 823 | $R^1$<br>$R^2$ | 2<br>2' | 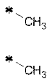 | 3 | 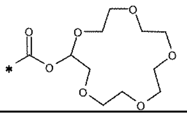 | 4 | 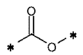 | | 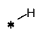 | 3' | | 4' | | | |
| | 14 | A-14 | 1234 | - | - | - | 3 | 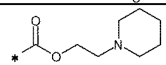 | 4 | 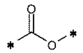 | | 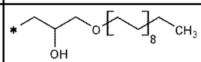 | 3' | | 4' | | | |
| | 15 | A-15 | 839 | - | - | - | 3 | 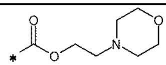 | 4 | 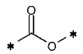 | | 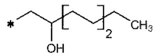 | 3' | | 4' | | | |
| | 16 | A-16 | 811 | - | - | - | 3 | 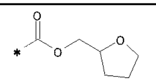 | 4 | 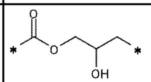 | 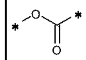 | 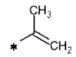 | 3' | | 4' | | | |
| | 17 | A-17 | 749 | - | - | - | 3 | 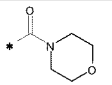 | 4 | 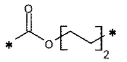 | 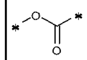 | 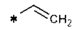 | 3' | | 4' | | | |
| | 18 | A-18 | 1212 | - | - | - | 3 | 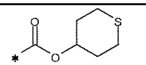 | 4 | 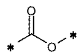 | | 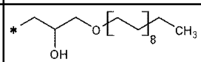 | 3' | | 4' | | | |
| | 19 | A-19 | 577 | - | - | - | 3 | 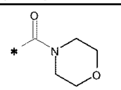 | 4 | 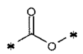 | | 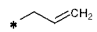 | 3' | | 4' | | | |
| | 20 | A-20 | 777 | - | - | - | 3 | 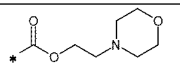 | 4 | 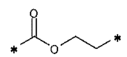 | 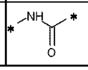 | 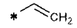 | 3' | | 4' | | | |

* is a connecting site.

$R^1$ to $R^4$, $Q^2$-$L^1$-$R^5$, $Q^4$-$L^2$-$R^6$, $Q^3$: - represents a hydrogen atom.

$L^1$, $L^2$: In the absence of description, a direct bond is represented.

Example 21: Synthesis of photopolymerization initiator (A-21)

To a 300 mL capacity flask equipped with a reflux condenser tube, a stirrer, a thermometer, and a dropping funnel, 79.27 g of (A-2) and 0.05 g of dibutyl hydroxytoluene (BHT) as a polymerization inhibitor were added, and the temperature was raised to 70°C while stirring. Thereafter, 20.73 g of (C1-1) and 0.02 g of dibutyltin dilaurate were added to the mixed liquid, the mixture was reacted at 70°C for 5 hours, disappearance of an absorption peak of an isocyanate group was confirmed by IR analysis, the reaction was completed, and purification was performed to obtain a pale yellow liquid. By IR analysis of the obtained liquid, the N-H specific absorption (1532 $cm^{-1}$) of a urethane bond and the C=O specific absorption (1650 $cm^{-1}$) of a ketone group of benzophenone were confirmed, and the molecular weight obtained by LC-MS analysis was 681, which was consistent with the molecular weight of the target compound shown in Table 3, and generation of a photopolymerization initiator (A-21) was confirmed.

Example 22: Synthesis of photopolymerization initiator (A-22)

Using an apparatus similar to that in Example 21, 34.42 g of (C1-2) and 0.02 g of dibutyltin dilaurate were added to a mixed liquid of 47.59 g of (A-6) and 0.05 g of BHT at 70°C, and the mixture was reacted at 70°C for 5 hours. After confirming by IR analysis that the reduction of isocyanate groups had stopped, 17.99 g of (B3-1) and 0.01 g of dibutyltin dilaurate were added, and the mixture was further reacted at 70°C for 3 hours. After the reaction was completed, disappearance of the isocyanate groups was confirmed by IR analysis, and purification was performed to obtain a pale yellow solid. Similarly, by IR analysis of the solid, the presence of a urethane group (1532 $cm^{-1}$) and a benzophenone group (1650 $cm^{-1}$) was confirmed, and generation of a photopolymerization initiator (A-22) was confirmed. The number average molecular weight of (A-22) was calculated to be 1,900 by GPC analysis, and the results are shown in Table 3.

Example 23: Synthesis of photopolymerization initiator (A-23)

Using an apparatus similar to that in Example 21, 20.47 g of (A-7), 31.95 g of (B1-1), 30 g of (H-1), and 0.05 g of BHT were mixed at 70°C, then 13.90 g of (C1-3) and 0.02 g of dibutyltin dilaurate were then added, the mixture was reacted at 70°C for 4 hours, and stop of reduction of isocyanate groups was confirmed by IR analysis. Thereafter, 3.68 g of (B3-2) and 0.01 g of dibutyltin dilaurate were added to the reaction solution, and the mixture was reacted at 70°C for 4 hours. Disappearance of the isocyanate groups was confirmed by IR analysis, and purification was performed to obtain a pale yellow viscous liquid. Similarly, the generation of a photopolymerization initiator (A-23) was confirmed by IR analysis. The number average molecular weight of (A-23) was calculated to be 6,600 by GPC analysis, and the results are shown in Table 3.

Examples 24 to 27, 29, and 30: Synthesis of photopolymerization initiators (A-24) to (A-27), (A-29), and (A-30)

Synthesis, purification, and analysis were performed in the same manner as in Example 23 with the composition shown in Table 3 to confirm generation of photopolymerization initiators (A-24) to (A-27), (A-29), and (A-30). The number average molecular weight calculated by GPC analysis is shown in Table 3.

Example 28: Synthesis of photopolymerization initiator (A-28)

Synthesis, purification, analysis, and the like were performed in the same manner as in Example 23 with the composition shown in Table 3 to confirm generation of a photopolymerization initiator (A-28). The number average molecular weight of (A-28) was calculated to be 10,500 by GPC analysis, and the results are shown in Table 3.

Example 31: Synthesis of photopolymerization initiator (A-31)

A nitrogen introduction tube was attached to an apparatus similar to that in Example 21, 53.04 g of (A-11), 9.37 g of (B1-9), 28.97 g of (C2-1), and 0.05 g of BHT were mixed, and the temperature was raised to 190°C while nitrogen was blown into the mixed liquid under normal pressure. To the mixed liquid, 0.01 g of zinc oxide was added, and a reaction was performed at 195°C while distilling out water. After the distillation out of water was stopped, the acid value (in accordance with JIS K0070:1992) of the reaction solution was measured and confirmed to be 48 mgKOH/g. Thereafter, the reflux condenser was changed to a Dean-Stark type azeotropic fractionation apparatus, 4.57 g of (B3-2), 4.05 g of (B3-8), and 0.01 g of concentrated sulfuric acid were added to the reaction solution, and both water and toluene (azeotropically were distilled out of the reaction system at 125°C. After the reaction was completed, the reaction solution was purified to obtain a pale yellow viscous liquid. Similarly, the generation of a photopolymerization initiator (A-31) was confirmed by IR analysis, and the number average molecular weight was calculated to be 3,800 by GPC analysis (Table 3).

Example 32: Synthesis of photopolymerization initiator (A-32)

Using an apparatus similar to that in Example 21, 40.23 g of (A-7), 12.56 g of (B2-1), 15 g of (H-5), and 0.05 g of BHT were mixed at 70°C. To the mixed liquid, 21.86 g of (C1-3) was added, and the mixture was reacted at 70°C for 2 hours. After confirming by IR analysis that the reduction of isocyanate groups had stopped, 4.53 g of (B3-3), 5.82 g of (B3-9), and 0.01 g of dibutyltin dilaurate were added, and the mixture was reacted at 70°C for 3 hours. After confirming by IR analysis that the isocyanate groups had disappeared, purification was performed to obtain a pale yellow viscous liquid. Similarly, the generation of a photopolymerization initiator (A-32) was confirmed by IR analysis, and the number average molecular weight was calculated to be 4,100 by GPC analysis (Table 3).

Example 33: Synthesis of photopolymerization initiator (A-33)

Using an apparatus similar to that in Example 31, 19.44 g of (A-10), 18.40 g of (C2-2), 15 g of (H-5), and 0.05 g of BHT were added and mixed. To the mixed liquid, 40.42 g of (C1-2) and 0.02 g of dibutyltin dilaurate were added, and the mixture was heated to 120°C over 2 hours and further reacted at 120°C for 10 hours. Thereafter, the reaction solution was cooled to 60°C, 5.28 g of (B3-1), 1.46 g of (B3-6), and 0.01 g of dibutyltin dilaurate were added thereto, and the mixture was reacted at 60°C for 2 hours. After the reaction was completed, disappearance of isocyanate groups was confirmed by IR analysis, and further purification was performed to obtain a pale yellow viscous liquid. Similarly, the generation of a photopolymerization initiator (A-33) was confirmed by IR analysis, and the number average molecular weight was calculated to be 2,800 by GPC analysis (Table 3).

[Table 3]

| | | Photo-polymerization initiator (A) | Compound of general formula (1) | | Polyol or amine compound | | Isocyanate compound | | Carboxylic acid compound | | Monoalcohol compound | | Organic solvent | | Number average molecular weight |
|---|---|---|---|---|---|---|---|---|---|---|---|---|---|---|---|
| | | | (A) | (g) | (B1) (B2) | (g) | (C1) | (g) | (C2) | (g) | (B3) | (g) | (H) | (g) | Mn |
| Examples | 21 | A-21 | A-2 | 79.27 | - | - | C1-1 | 20.73 | - | - | - | - | - | - | 681 |
| | 22 | A-22 | A-6 | 47.59 | - | - | C1-2 | 34.42 | - | - | B3-1 | 17.99 | - | - | 1,900 |
| | 23 | A-23 | A-7 | 20.47 | B1-1 | 31.95 | C1-3 | 13.90 | - | - | B3-2 | 3.68 | H-1 | 30 | 6,600 |
| | 24 | A-24 | A-9 | 38.02 | B1-2 | 22.05 | C1-4 | 6.75 | - | - | B3-3 | 3.18 | H-2 | 30 | 9,500 |
| | 25 | A-25 | A-11 | 27.16 | B1-3 | 18.98 | C1-2 | 18.05 | - | - | B3-4 | 5.81 | H-1 | 30 | 5,100 |
| | 26 | A-26 | A-14 | 15.81 | B1-4 | 38.43 | C1-5 | 12.44 | - | - | B3-2<br>B3-3 | 1.47<br>1.85 | H-3 | 30 | 8,200 |
| | 27 | A-27 | A-15 | 26.06 | B1-5 | 15.49 | C1-5 | 7.89 | - | - | B3-3 | 0.56 | H-4 | 50 | 38,700 |
| | 28 | A-28 | A-18 | 17.33 | B1-6 | 71.53 | C1-3 | 7.47 | - | - | B3-2<br>B3-5 | 1.65<br>2.02 | - | - | 10,500 |
| | 29 | A-29 | A-11 | 42.82 | B1-7 | 4.80 | C1-2 | 21.35 | - | - | B3-6 | 1.03 | H-4 | 30 | 6,600 |
| | 30 | A-30 | A-6 | 18.55 | B1-8 | 5.61 | C1-6 | 28.75 | - | - | B3-3<br>B3-7 | 5.59<br>1.50 | H-1 | 40 | 20,700 |
| | 31 | A-31 | A-11 | 53.04 | B1-9 | 9.37 | - | - | C2-1 | 28.97 | B3-2<br>B3-8 | 4.57<br>4.05 | - | - | 3,800 |
| | 32 | A-32 | A-7 | 40.23 | B2-1 | 12.56 | C1-3 | 21.86 | - | - | B3-3<br>B3-9 | 4.53<br>5.82 | H-5 | 15 | 4,100 |
| | 33 | A-33 | A-10 | 19.44 | - | - | C1-2 | 40.42 | C2-2 | 18.40 | B3-1<br>B3-6 | 5.28<br>1.46 | H-5 | 15 | 2,800 |

Examples 34 to 72 and Comparative Examples 1 to 4

Using the photopolymerization initiators (A-1) to (A-33) obtained in Examples and a known photopolymerization initiator (D), a monofunctional monomer (E), a polyfunctional monomer or oligomer (F), and other components (G) were weighed in the proportion shown in Table 4, and mixed at 25°C for 30 minutes to prepare an active energy ray curable composition. The transparency of the obtained active energy ray curable composition, the compatibility of the photopolymerization initiator (A) or (D) with the monofunctional monomer (E) or with the polyfunctional monomer or oligomer (F), and the curability of the curable composition under different curing conditions (presence or absence of oxygen inhibition, active energy rays having different wavelengths) were evaluated by the following methods, and the results thereof are

shown in Table 4-1 and Table 4-2.

(5-1) Transparency and compatibility

The state of the obtained active energy ray curable composition was visually observed, and the transparency was evaluated according to the following criteria. In addition, 20 g of each of the obtained photopolymerization initiators (A-1) to (A-33) and known photopolymerization initiators (D-1) to (D-4) and 80 g of each of the monofunctional monomers (E-2) and (E-3), or the polyfunctional monomer or oligomer (F-15) were weighed and mixed at 25°C for 30 minutes to prepare evaluation liquids. The state of the mixed liquid was visually observed, and the compatibility of the photopolymerization initiator with the monofunctional monomer or with the polyfunctional monomer or oligomer was evaluated according to the following criteria. The evaluation of transparency and the evaluation of compatibility were performed according to the same criteria.

○: High transparency or compatibility, and no turbidity or separation was observed.
△: No phase separation was observed, but turbidity was observed.
×: Turbidity or phase separation was observed.

(5-2) Curability under conditions without oxygen inhibition

The obtained active energy ray curable composition was applied onto an adhesionfacilitating treated surface of a polyethylene terephthalate (PET) film (COSMOSHINE A-4100, manufactured by Toyobo Co., Ltd.) with a thickness of 100 μm. Using a bar coater to make a coating film with a thickness of 20 μm, and then a release surface of a biaxially stretched PET film (DIAFOIL MRF38, manufactured by Mitsubishi Plastics, Inc.) having a release surface on one side was bonded to the applied surface of the coating film. Thereafter, the coating film was cured by irradiation with ultraviolet rays, and a cumulative amount of light at which there is no tack at the time of contact with the cured product by flipping the release surface was obtained, and curability was evaluated in four grades. The following two types of lamps 1) and 2) were used for ultraviolet irradiation. In addition, the lower the cumulative amount of light required until the tack disappears, the higher the curability.

1) UV-LED lamp: wavelength 365 nm, output power 100 $mW/cm^2$
2) UV-LED lamp: wavelength 405 nm, output power 100 $mW/cm^2$

⊙: When the cumulative amount of light was less than 1000 $mJ/cm^2$, there is no tack.
○: When the cumulative amount of light was 1000 $mJ/cm^2$ or more or less than 3000 $mJ/cm^2$, there is no tack.
△: When the cumulative amount of light was 3000 $mJ/cm^2$ or more and less than 20000 $mJ/cm^2$, there is no tack.
×: Tack remained even at the cumulative amount of light of 20000 $mJ/cm^2$.

(5-3) Curability under conditions with oxygen inhibition

A coating film before curing was prepared in the same manner as in the curability evaluation under conditions without oxygen inhibition, a film was not bonded to the surface of the coating film, the coating film was cured by irradiation with ultraviolet rays, a cumulative amount of light at which there is no tack at the time of contact with the cured product was similarly obtained, and curability was evaluated in four grades in the same manner.

(5-4) Bleed-out resistance

A test piece after curing was prepared in the same manner as in the curability evaluation under conditions without oxygen inhibition (UV-LED lamp: wavelength 395 nm, illumination 100 $mW/cm^2$, cumulative amount of light 10,000 $mJ/cm^2$), the test piece was allowed to stand for 168 hours in a thermo-hygrostat bath set at a temperature of 40°C and a relative humidity of 50%, the surface of the test piece was visually observed, and the bleed-out resistance was evaluated according to the following criteria.

⊙: No bleed-out was observed.
○: Bleed-out was very slightly observed.
△: Bleed-out was slightly observed.
×: Bleed-out was severely observed.

[Table 4-1]

| | | Active energy ray curable composition (% by mass) | | | | | | | | Transparency | Compatibility | | | Curability (UV-LED) | | | | Bleed-out resistance |
|---|---|---|---|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | E-2 | E-3 | F-15 | Without oxygen inhibition 365 nm | Without oxygen inhibition 405 nm | With oxygen inhibition 365 nm | With oxygen inhibition 405 nm | |
| | | | | | | | | | | | | | | 100 mW/cm$^2$ | | 1,000 mW/cm$^2$ | | |
| Examples | 34 | A-1 | 20 | E-1<br>E-2<br>E-3 | 20<br>20<br>30 | F-1 | 10 | - | - | ○ | ○ | ○ | ○ | ○ | △ | ○ | △ | ○ |
| | 35 | A-2 | 10 | E-4<br>E-5 | 40<br>30 | F-2 | 20 | - | - | ○ | △ | ○ | ○ | ○ | △ | ○ | △ | ○ |
| | 36 | A-3 | 20 | E-3<br>E-5<br>E-6 | 25<br>30<br>5 | F-1<br>F-2 | 10<br>10 | - | - | ○ | ○ | ○ | ○ | ○ | △ | △ | △ | ○ |
| | 37 | A-4 | 5 | E-3 | 40 | F-3 | 50 | G-2 | 5 | ○ | ○ | ○ | ○ | ○ | △ | △ | △ | × |
| | 38 | A-5 | 20 | E-4<br>E-7<br>E-8 | 25<br>10<br>25 | F-4 | 20 | - | - | ○ | ○ | ○ | ○ | ○ | △ | △ | △ | ○ |
| | 39 | A-6 | 10 | E-3<br>E-9<br>E-10 | 30<br>10<br>10 | F-5<br>F-6 | 20<br>20 | - | - | ○ | △ | ○ | △ | ○ | ○ | ○ | △ | △ |
| | 40 | A-7 | 9 | E-1<br>E-3<br>E-5<br>E-6 | 20<br>20<br>30<br>20 | F-7 | 1 | - | - | ○ | △ | ○ | ○ | ○ | ○ | ○ | ○ | △ |
| | 41 | A-8 | 5 | E-2<br>E-8<br>E-11 | 20<br>30<br>5 | F-1<br>F-8 | 30<br>5 | G-1 | 5 | ○ | ○ | ○ | ○ | ○ | ○ | ○ | △ | △ |
| | 42 | A-9 | 20 | - | - | F-9<br>F-10 | 15<br>65 | - | - | ○ | ○ | ○ | ○ | ◎ | ○ | ○ | ○ | ○ |
| | 43 | A-10 | 5 | E-1 | 45 | F-4<br>F-10 | 10<br>40 | - | - | ○ | △ | ○ | ○ | ○ | ○ | ○ | ○ | △ |
| | 44 | A-11 | 0.5 | E-4<br>E-8<br>E-12 | 30<br>15<br>20 | F-1<br>F-11 | 20<br>10 | G-2 | 4.5 | ○ | △ | ○ | △ | ○ | ○ | ○ | ○ | △ |
| | 45 | A-12 | 10 | E-2<br>E-10 | 20<br>30 | F-2 | 40 | - | - | ○ | ○ | ○ | ○ | ○ | ○ | ○ | ○ | △ |
| | 46 | A-12 | 5 | E-3 | 50 | F-1<br>F-10 | 30<br>10 | G-1 | 5 | ○ | ○ | ○ | ○ | ○ | ○ | ○ | ○ | ○ |
| | 47 | A-13 | 5 | E-8 | 40 | F-2<br>F-12<br>F-13 | 25<br>25<br>5 | - | - | ○ | △ | ○ | ○ | ○ | ○ | ○ | △ | △ |
| | 48 | A-14 | 25 | E-10 | 5 | F-5<br>F-14<br>F-10 | 15<br>25<br>30 | - | - | ○ | ○ | ○ | ○ | ◎ | ○ | ◎ | ○ | ○ |
| | 49 | A-15 | 10 | E-5 | 40 | F-15<br>F-16 | 30<br>20 | - | - | ○ | △ | ○ | ○ | ◎ | ◎ | ◎ | ○ | ○ |
| | 50 | A-16 | 5 | E-1<br>E-3<br>E-5<br>E-12 | 35<br>5<br>10<br>5 | F-1<br>F-2 | 20<br>20 | - | - | ○ | ○ | ○ | ○ | ◎ | ○ | ◎ | ○ | ◎ |
| | 51 | A-16 | 0.5 | E-2<br>E-3 | 20<br>20 | F-2<br>F-14 | 50.5<br>9 | - | - | ○ | ○ | ○ | ○ | ○ | ○ | ○ | △ | ◎ |
| | 52 | A-17 | 2 | E-4 | 40 | F-1<br>F-7<br>F-3 | 43<br>5<br>10 | - | - | ○ | ○ | ○ | ○ | ◎ | ○ | ◎ | ○ | ◎ |
| | 53 | A-17 | 40 | E-2 | 10 | F-2<br>F-11 | 30<br>15 | G-2 | 5 | ○ | ○ | ○ | ○ | ◎ | ◎ | ◎ | ◎ | ◎ |
| | 54 | A-18 | 3 | E-4<br>E-10 | 40<br>32 | F-1<br>F-12 | 15<br>10 | - | - | ○ | ○ | ○ | ○ | ○ | ○ | ○ | △ | ○ |
| | 55 | A-19 | 5 | E-1<br>E-4<br>E-11 | 20<br>20<br>5 | F-9<br>F-10 | 20<br>30 | - | - | ○ | ○ | ○ | ○ | ◎ | ◎ | ◎ | ○ | ◎ |
| | 56 | A-19 | 0.1 | E-3<br>E-8 | 50<br>35 | F-8 | 14 | G-2 | 0.9 | ○ | ○ | ○ | ○ | ○ | △ | △ | △ | ◎ |
| | 57 | A-20 | 50 | E-5<br>E-8 | 25<br>10 | F-15<br>F-17 | 10<br>5 | - | - | ○ | ○ | ○ | ○ | ◎ | ◎ | ◎ | ◎ | ◎ |
| | 58 | A-20 | 1 | E-1<br>E-10<br>E-13 | 30<br>30<br>10 | F-14 | 29 | - | - | ○ | ○ | ○ | ○ | ◎ | ○ | ◎ | ○ | ◎ |

[Table 4-2]

| | | Active energy ray curable composition (% by mass) | | | | | | | | Transparency | Compatibility | | | Curability (UV-LED) | | | | Bleed-out resistance |
|---|---|---|---|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | E-2 | E-3 | F-15 | Without oxygen inhibition 365 nm 100 mW/cm$^2$ | Without oxygen inhibition 405 nm 100 mW/cm$^2$ | With oxygen inhibition 365 nm 1,000 mW/cm$^2$ | With oxygen inhibition 405 nm 1,000 mW/cm$^2$ | |
| Examples | 59 | A-21 | 5 | E-3<br>E-6 | 40<br>10 | F-5<br>F-3<br>F-12 | 20<br>20<br>5 | - | - | ○ | ○ | ○ | ○ | ○ | ○ | ○ | △ | ◎ |
| | 60 | A-22 | 0.5 | E-4<br>E-8 | 5<br>45 | F-2<br>F-10 | 20<br>25 | G-2 | 4.5 | ○ | ○ | ○ | ○ | ○ | ○ | ○ | △ | ◎ |
| | 61 | A-23 | 70 | E-10 | 25 | F-1 | 5 | - | - | ○ | ○ | ○ | ○ | ◎ | ◎ | ◎ | ◎ | ◎ |
| | 62 | A-23 | 100 | - | - | - | - | - | - | ○ | ○ | ○ | ○ | ◎ | ◎ | ◎ | ◎ | ◎ |
| | 63 | A-24 | 30 | E-3<br>E-13 | 30<br>20 | F-10<br>F-17 | 10<br>10 | - | - | ○ | ○ | ○ | ○ | ◎ | ◎ | ◎ | ◎ | ◎ |
| | 64 | A-25 | 10 | E-3 | 40 | F-10 | 50 | - | - | ○ | ○ | ○ | ○ | ◎ | ◎ | ◎ | ○ | ◎ |
| | 65 | A-26 | 10 | E-1<br>E-3<br>E-10 | 20<br>15<br>20 | F-3<br>F-14<br>F-17 | 5<br>15<br>15 | - | - | ○ | ○ | ○ | ○ | ◎ | ◎ | ◎ | ○ | ◎ |
| | 66 | A-27 | 50 | E-2<br>E-8 | 20<br>30 | - | - | - | - | ○ | ○ | ○ | ○ | ◎ | ◎ | ◎ | ◎ | ◎ |
| | 67 | A-28 | 10 | E-8 | 30 | F-1<br>F-9<br>F-6 | 10<br>20<br>30 | - | - | ○ | ○ | ○ | ○ | ◎ | ○ | ○ | ○ | ◎ |
| | 68 | A-29 | 1 | E-5<br>E-14 | 60<br>10 | F-2 | 28 | G-2 | 1 | ○ | ○ | ○ | ○ | ○ | ○ | ○ | △ | ◎ |
| | 69 | A-30 | 10 | E-1<br>E-2<br>E-15 | 30<br>20<br>5 | F-1<br>F-2 | 15<br>20 | - | - | ○ | ○ | ○ | ○ | ◎ | ○ | ○ | ○ | ◎ |
| | 70 | A-31 | 20 | E-5 | 40 | F-11<br>F-14 | 15<br>20 | G-2 | 5 | ○ | ○ | ○ | ○ | ◎ | ◎ | ◎ | ○ | ◎ |
| | 71 | A-32 | 3 | E-8 | 2 | F-1<br>F-5<br>F-17 | 40<br>25<br>30 | - | - | ○ | ○ | ○ | ○ | ○ | ○ | ○ | ○ | ◎ |
| | 72 | A-33 | 5 | E-3 | 45 | F-1 | 50 | - | - | ○ | ○ | ○ | ○ | ○ | ○ | ○ | ○ | ◎ |
| Comparative Examples | 1 | D-1 | 5 | E-3<br>E-4 | 25<br>20 | F-1 | 50 | - | - | ○ | ○ | ○ | ○ | ○ | × | △ | × | × |
| | 2 | D-1 | 0.5 | E-2<br>E-8 | 15<br>20 | F-16 | 59.5 | G-2 | 5 | ○ | ○ | ○ | ○ | ◎ | × | ○ | × | △ |
| | 3 | D-2 | 10 | E-5 | 40 | F-2<br>F-10 | 20<br>30 | - | - | ○ | △ | ○ | ○ | ○ | × | △ | × | × |
| | 4 | D-3 | 10 | E-8<br>E-11 | 30<br>20 | F-14 | 40 | - | - | ○ | △ | ○ | ○ | ○ | △ | △ | × | × |

Examples 73 to 79 and Comparative Examples 5 to 7

Using the obtained photopolymerization initiator (A) and a known photopolymerization initiator (D), a monofunctional monomer (E), a polyfunctional monomer or oligomer (F), other components (G), and an organic solvent (H) were weighed in the proportions shown in Table 5, and then mixed at 25°C for 30 minutes to prepare an active energy ray curable coating composition. Adhesion, pencil hardness, and durability of the coating composition were evaluated by the following methods, and the results are shown in Table 5.

(5-5) Adhesion

The obtained coating composition was applied onto each test piece of a PET plate (PET-1060, manufactured by C.I. TAKIRON Corporation), a polycarbonate (PC) plate (PC1600, manufactured by C.I. TAKIRON Corporation), a glass (GL) plate (EAGLE XG, manufactured by Corning Japan K.K.), and a SUS 304 plate using a bar coater so as to have a dry film thickness of 10 μm, and dried in a thermostatic bath at 80°C for 2 minutes. Irradiation was performed using a UV-LED lamp with a wavelength of 395 nm and an output power of 100 mW/cm$^2$ so that the cumulative amount of light was 3000 mJ/cm$^2$ to prepare a cured film. Using the obtained cured film, 100 squares of 1 mm$^2$ were prepared with a cutter knife according to JIS K 5600-5-6, and the number of squares remaining on the test piece when a commercially available

pressure-sensitive adhesive tape was bonded and then peeled off was evaluated in four grades. The greater the number of squares remaining on the test piece, the higher the adhesion.

⊙: The number of remaining squares was 100.
○: The number of remaining squares was 90 to 99.
△: The number of remaining squares was 60 to 89.
×: The number of remaining squares was less than 60.

(5-6) Pencil hardness

Using a cured film prepared on a test piece of PC in the same manner as in the adhesion evaluation, the surface of the cured film was scratched with a pencil (angle of 45°, about 10 mm) according to JIS K 5600-5-4, and then the hardest pencil without scratching the surface of the cured film was defined as pencil hardness, and evaluation was performed in 4 grades.

⊙: Pencil hardness was 2H or more.
○: Pencil hardness was HB to H.
△: Pencil hardness was 3B to B.
×: Pencil hardness was 4B or less.

(5-7) Durability

Using a cured film prepared on a test piece of PC in the same manner as in the adhesion evaluation, after holding for 100 hours under conditions of a temperature of 85°C and a relative humidity of 85%, the presence or absence of lifting or peeling of the cured film, generation of bubbles or cloudiness was visually observed, and the durability was evaluated according to the following criteria.

⊙: It was transparent, and no lifting, peeling, or bubbles.
○: It had very slight cloudiness, but no lifting, peeling, or bubbles.
△: Lifting, peeling, or bubbles were slightly generated.
×: Cloudiness, lifting, peeling, or bubbles were present.

[Table 5]

| | | Active energy ray curable coating composition (% by mass) | | | | | | | | | | Adhesion | | | | Pencil hardness | Durability |
|---|---|---|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | Organic solvent (H) | | PET | PC | GL | SUS304 | | |
| Examples | 73 | A-1 | 4 | E-3<br>E-5 | 12<br>8 | F-5<br>F-7 | 8<br>8 | - | - | H-3 | 60 | ◎ | ○ | ○ | ○ | ○ | ○ |
| | 74 | A-8 | 3 | E-3<br>E-6 | 30<br>40 | F-7<br>F-9 | 10<br>17 | - | - | - | - | ◎ | ◎ | ○ | ○ | ○ | ○ |
| | 75 | A-12 | 1 | E-3 | 39 | F-5<br>F-11 | 40<br>20 | - | - | - | - | ◎ | ◎ | ○ | ○ | ◎ | ○ |
| | 76 | A-17 | 10 | E-3<br>E-5 | 30<br>20 | F-5<br>F-7 | 20<br>10 | G-3 | 10 | - | - | ◎ | ◎ | ○ | ○ | ◎ | ○ |
| | 77 | A-23 | 20 | E-7<br>E-8 | 5<br>15 | F-9 | 10 | - | - | H-1 | 50 | ◎ | ◎ | ◎ | ○ | ◎ | ○ |
| | 78 | A-26 | 20 | E-3<br>E-6 | 30<br>40 | F-7 | 10 | - | - | - | - | ◎ | ◎ | ◎ | ◎ | ○ | ○ |
| | 79 | A-30 | 10 | E-3 | 30 | F-5<br>F-7 | 35<br>20 | G-3 | 5 | - | - | ◎ | ◎ | ◎ | ◎ | ◎ | ○ |
| Comparative Examples | 5 | D-2 | 5 | E-3<br>E-5 | 30<br>20 | F-5<br>F-7 | 25<br>20 | - | - | - | - | △ | △ | × | × | △ | △ |
| | 6 | D-3 | 10 | E-3<br>E-5 | 30<br>20 | F-5<br>F-7 | 20<br>10 | G-3 | 10 | - | - | ○ | ○ | △ | × | ○ | × |
| | 7 | D-4 | 40 | E-7<br>E-8 | 10<br>30 | F-9 | 20 | - | - | - | - | × | × | × | × | × | △ |

Examples 80 to 85 and Comparative Examples 8 and 9

Using the obtained photopolymerization initiator (A) and a known photopolymerization initiator (D), a monofunctional monomer (E), a polyfunctional monomer or oligomer (F), and other components (G) were weighed in the proportions shown in Table 6, and then mixed at 25°C for 30 minutes to prepare an active energy ray curable ink composition. The obtained ink composition was applied to a PET film having a thickness of 100 μm by a bar coater (RDS12) (film thickness after drying: 20 μm), and cured by ultraviolet ray irradiation (UV-LED lamp: wavelength 395 nm, illumination 1000 mW/cm$^2$) to prepare a printed matter. The curability of the ink composition was evaluated in the same manner as in the evaluation of curability under the conditions with oxygen inhibition in (5-3) above, and the viscosity of the ink composition, pigment dispersibility, ejection stability, and print sharpness of the printed matter were evaluated by the following methods, and the results are shown in Table 6.

(5-8) Viscosity

The viscosity of the ink composition was measured with a cone-plate viscometer (RE 550 type viscometer, manufactured by Toki Sangyo Co., Ltd.) at 25°C in accordance with JIS K 5600-2-3, and evaluated as an ink composition for inkjet printing in the following 4 grades.

⊙: Less than 5 to 50 mPa·s
○: Less than 50 to 500 mPa·s
△: Less than 500 to 2000 mPa·s
×: 2000 mPa·s or more

(5-9) Pigment dispersibility

Using an ink composition containing a pigment, the state of aggregation or precipitation of the pigment was visually observed immediately after preparation and after standing at room temperature for 2 months, and the pigment dispersibility was evaluated according to the following criteria.

⊙: Neither aggregation nor precipitation of the pigment was observed immediately after preparation nor after standing for 2 months.
○: Neither aggregation nor precipitation was observed immediately after preparation, but slight aggregation or precipitation of the pigment was observed after standing for 2 months.
△: Slight aggregation or precipitation was observed immediately after preparation, and aggregation or precipitation of the pigment was clearly observed after standing for 2 months.
×: Aggregation or precipitation of the pigment was clearly observed even immediately after preparation.

(5-10) Ejection stability

The obtained ink composition was filled into an inkjet printer (LuxelJet UV3 50GTW, manufactured by FUJIFILM Corporation), a solid image was printed using coated paper, the printing state of the obtained printed matter was visually observed, and the ejection stability was evaluated according to the following criteria.

⊙: Good printing was performed without nozzle omission.
○: Slight nozzle omission was observed.
×: There was nozzle omission in a wide range.

(5-11) Print sharpness

The image clarity of the printed matter obtained in the ejection stability evaluation was visually observed and evaluated according to the following criteria.

⊙: Ink bleeding was not observed at all, and the image was clear.
○: There was almost no ink bleeding, and the image was good.
×: Ink bleeding was observed.

[Table 6]

| | | Active energy ray curable ink composition (% by mass) | | | | | | | | Viscosity | Curability | Pigment dispersibility | Ejection stability | Print sharpness |
|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | | | | |
| Examples | 80 | A-2 | 20 | E-3 | 30 | F-5 | 45 | G-4<br>G-5 | 4<br>1 | ◎ | ○ | ◎ | ○ | ◎ |
| | 81 | A-3 | 30 | E-4<br>E-9 | 30<br>10 | F-5<br>F-6 | 20<br>5 | G-4 | 5 | ◎ | ○ | - | ○ | - |
| | 82 | A-5 | 20 | E-2 | 25 | F-5 | 52.5 | G-5 | 2.5 | ○ | ○ | ◎ | ◎ | ◎ |
| | 83 | A-16 | 9 | E-5 | 27 | F-5 | 45 | G-4<br>G-6 | 9<br>10 | ○ | ○ | ○ | ◎ | ○ |
| | 84 | A-20 | 5 | E-2<br>E-8 | 25<br>15 | F-5 | 52.5 | G-4<br>G-6 | 2<br>0.5 | ◎ | ◎ | ◎ | ◎ | ◎ |
| | 85 | A-22 | 6 | E-1<br>E-4<br>E-9 | 24<br>40<br>12 | F-6 | 18 | - | - | ◎ | ○ | - | ◎ | - |
| Comparative Examples | 8 | D-1 | 6 | E-1<br>E-4<br>E-9 | 24<br>40<br>12 | F-6 | 18 | - | - | ◎ | × | - | ◎ | - |
| | 9 | D-5 | 20 | E-2 | 25 | F-5 | 52.5 | G-5 | 2.5 | ○ | ○ | × | × | × |

Examples 86 to 90 and Comparative Examples 10 and 11

Using the obtained photopolymerization initiator (A) and a known photopolymerization initiator (D), a monofunctional monomer (E), a polyfunctional monomer or oligomer (F), and other components (G) were weighed in the proportion shown in Table 7, and mixed at 25°C for 30 minutes to prepare an active energy ray curable pressure-sensitive adhesive composition. A 75 μm-thick heavy-release PET film (E7001, manufactured by Toyobo Co., Ltd.) was brought into close contact with a horizontally placed glass plate, a spacer with a thickness of 1 mm and an internal size of 60 mm × 100 mm was placed, the prepared pressure-sensitive adhesive composition of Examples and Comparative Examples was filled inside the spacer, a 50 μm-thick light-release PET film (E7002, manufactured by Toyobo Co., Ltd.) was placed thereon, and irradiation was performed using a UV-LED lamp with a wavelength of 395 nm and an illumination of 100 $mW/cm^2$ so that the cumulative amount of light was 1000 $mJ/cm^2$ to cure the pressure-sensitive adhesive composition. Thereafter, the light-release PET film was peeled off to obtain a pressure-sensitive adhesive sheet including a cured product (pressure-sensitive adhesive layer) of the pressure-sensitive adhesive composition and a heavy-release PET film. The transparency, pressure-sensitive adhesive strength, reworkability, and yellowing resistance of the test piece were evaluated by the following methods, and the results are shown in Table 7.

(5-12) Transparency

The pressure-sensitive adhesive layer was transferred from the pressure-sensitive adhesive sheet to a glass plate under conditions of a temperature of 23°C and a relative humidity of 50%, and the transmittance of the glass plate and the pressure-sensitive adhesive layer was measured according to JIS K 7105 using a haze meter (NDH-2000, manufactured by Nippon Denshoku Industries Co., Ltd.). Thereafter, the transmittance of the glass plate was measured in the same manner, subtracted from the total light transmission of the glass plate and the pressure-sensitive adhesive layer, the transmission of the pressure-sensitive adhesive layer was calculated, and the transparency was evaluated according to the following criteria.

⊙: Transmission was 90% or more.
○: Transmission was 85% or more and less than 90%.
△: Transmission was 50% or more and less than 85%.
×: Transmission was less than 50%.

(5-13) Yellowing resistance

A pressure-sensitive adhesive sheet was prepared in the same manner as above, set in a xenon fade meter (SC-700-WA, manufactured by Suga Test Instruments Co., Ltd.), and irradiated with ultraviolet rays with an intensity of 70 $mW/cm^2$ for 120 hours, and then visually observed for discoloration of the pressure-sensitive adhesive layer on the pressure-sensitive adhesive sheet. The evaluation was performed according to the following criteria.

⊙: Yellowing could not be visually observed at all.
○: Very slight yellowing could be visually observed.
△: Yellowing could be visually observed.
×: Clear yellowing could be visually observed.

(5-14) Pressure-sensitive adhesive strength

The pressure-sensitive adhesive layer was transferred to the following substrate (film or plate) under conditions of a temperature of 23°C and a relative humidity of 50% for the pressure-sensitive adhesive sheet, and the substrate was pressure-bonded by moving back and forth twice using a pressure-bonding roller having a weight of 2 kg, and allowed to stand in the same atmosphere for 30 minutes. Thereafter, the 180° peel strength (N/25 mm) was measured at a peel speed of 300 mm/min according to JIS Z0237 using a tensile tester (TENSILON RTA-100, manufactured by ORIENTEC Co., Ltd.), and the pressure-sensitive adhesive strength of the pressure-sensitive adhesive sheet in each substrate was evaluated according to the following criteria.

PET: COSMOSHINE A4160 (corona-treated surface, manufactured by Toyobo Co., Ltd.)
PC: PC1600 (manufactured by C.I. TAKIRON Corporation)
GL (glass): EAGLE XG (manufactured by Corning Japan K.K.)
⊙: 20 (N/25 mm) or more
○: 10 (N/25 mm) or more and less than 20 (N/25 mm)
Δ: 5 (N/25 mm) or more and less than 10 (N/25 mm)
×: Less than 5 (N/25 mm)

(5-15) Reworkability

The pressure-sensitive adhesive layer was transferred from the pressure-sensitive adhesive sheet to the substrate in the same manner as in the evaluation of the pressure-sensitive adhesive strength. Thereafter, after allowing to stand in a thermostatic bath at 80°C for 24 hours, the remaining state of the pressure-sensitive adhesive layer (adhesive) on the surface of the substrate after peeling off the adhesive sheet was visually observed, and the reworkability of the pressure-sensitive adhesive sheet was evaluated according to the following criteria.

⊙: There was no adhesive residue.
○: There was very slight adhesive residue.
△: There was slight adhesive residue.
×: There was adhesive residue.

[Table 7]

| | | Active energy ray curable pressure-sensitive adhesive composition (% by mass) | | | | | | | | Transparency | Yellowing resistance | Pressure-sensitive adhesive strength | | | Rework-ability | | |
|---|---|---|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | | PET | PC | GL | PET | PC | GL |
| Examples | 86 | A-5 | 15 | E-3<br>E-5<br>E-7 | 20<br>20<br>5 | F-14 | 40 | - | - | ○ | ○ | ◎ | ○ | ○ | ◎ | ○ | ○ |
| | 87 | A-10 | 3 | E-2<br>E-11<br>E-12 | 10<br>5<br>40 | F-3 | 7 | G-7 | 35 | ○ | ○ | ◎ | ◎ | ◎ | ○ | ○ | ○ |
| | 88 | A-24 | 10 | E-3<br>E-5<br>E-10<br>E-11 | 10<br>40<br>25<br>5 | F-8 | 10 | - | - | ○ | ○ | ○ | ○ | ○ | ○ | ○ | ○ |
| | 89 | A-26 | 20 | E-5<br>E-7 | 50<br>5 | F-14 | 25 | - | - | ◎ | ◎ | ◎ | ◎ | ◎ | ○ | ○ | ○ |
| | 90 | A-27 | 40 | E-3<br>E-7<br>E-12 | 20<br>10<br>30 | - | - | - | - | ◎ | ◎ | ◎ | ◎ | ◎ | ◎ | ◎ | ◎ |
| Comparative Examples | 10 | D-5 | 20 | E-5<br>E-7 | 50<br>5 | F-14 | 25 | - | - | ○ | ○ | × | △ | × | × | △ | × |
| | 11 | D-4 | 3 | E-2<br>E-11<br>E-12 | 10<br>5<br>40 | F-3 | 7 | G-7 | 35 | ○ | ○ | ○ | △ | △ | △ | △ | × |

Examples 91 to 96 and Comparative Examples 12 and 13

Using the obtained photopolymerization initiator (A) and a known photopolymerization initiator (D), a monofunctional monomer (E), a polyfunctional monomer or oligomer (F), and other components (G) were weighed in the proportions shown in Table 8, and then mixed at 25°C for 30 minutes to prepare an active energy ray curable adhesive composition. The adhesive composition was applied onto various plate-shaped or film-shaped substrates placed horizontally, and the following PET film was bonded to the applied surface. Thereafter, bonding was performed using a tabletop roll laminator machine (RSL-382S) so as not to bite bubbles so that the adhesive layer had a thickness of 20 μm, and irradiation was performed using a UV-LED lamp with a wavelength of 405 nm and an illumination of 50 mW/cm$^2$ so that the cumulative amount of light was 2000 mJ/cm$^2$ to prepare a laminate. The adhesive strength and water resistance of the obtained laminate were evaluated by the following methods, and the results are shown in Table 8. The following substrates were used.

PET: Film-shaped (E5100, corona-treated surface, manufactured by Toyobo Co., Ltd.)
PMMA: Plate-shaped (COMOGLAS P, manufactured by Kuraray Co., Ltd.)
PC: Plate-shaped (PC1600, manufactured by C.I. TAKIRON Corporation)

(5-16) Water resistance

The prepared laminate was immersed in warm water at 60°C for 48 hours to confirm the presence or absence of peeling at the interface, and water resistance was evaluated according to the following criteria.

⊙: No peeling at interface (less than 1 mm).
○: Peeling was present at a part of interface (1 mm or more and less than 3 mm).
△: Peeling was present at a part of interface (3 mm or more and less than 5 mm).

×: Peeling was present at interface (5 mm or more).

(5-17) Adhesive strength

Regarding the prepared laminate, the 180° peel strength (N/25 mm) was measured at a peel speed of 300 mm/min according to JIS Z0237 using a tensile tester (TENSILON RTA-100), and the adhesive strength was evaluated according to the following criteria.

⊙: 20 (N/25 mm) or more
○: 10 (N/25 mm) or more and less than 20 (N/25 mm)
△: 5 (N/25 mm) or more and less than 10 (N/25 mm)
×: Less than 5 (N/25 mm)

[Table 8]

| | | Active energy ray curable adhesive composition (% by mass) | | | | | | | | Water resistance | Adhesive strength | | | Outgassing resistance |
|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | PET /PET | PMMA /PET | PC /PET | |
| Examples | 91 | A-4 | 3 | E-3<br>E-7 | 30<br>6 | F-7<br>F-6<br>F-15 | 10<br>30<br>20 | G-4 | 1 | ○ | ○ | △ | ○ | △ |
| | 92 | A-13 | 1 | E-3<br>E-7 | 50<br>20 | F-6 | 28 | G-4 | 1 | ○ | ○ | ○ | ○ | ○ |
| | 93 | A-20 | 5 | E-2<br>E-3<br>E-8 | 20<br>20<br>40 | F-7<br>F-15 | 5<br>10 | - | - | ◎ | ◎ | ○ | ○ | ◎ |
| | 94 | A-24 | 20 | E-2<br>E-5 | 40<br>20 | F-15 | 15 | G-3 | 5 | ○ | ○ | ○ | ○ | ◎ |
| | 95 | A-28 | 30 | E-7<br>E-8 | 10<br>60 | - | - | - | - | ○ | ○ | ○ | ○ | ◎ |
| | 96 | A-30 | 20 | E-2<br>E-3 | 40<br>20 | F-7 | 20 | - | - | ◎ | ◎ | ◎ | ◎ | ◎ |
| Comparative Examples | 12 | D-3 | 1 | E-3<br>E-7 | 50<br>20 | F-6 | 28 | G-4 | 1 | × | △ | × | △ | △ |
| | 13 | D-5 | 20 | E-2<br>E-3 | 40<br>20 | F-7 | 20 | - | - | × | △ | △ | △ | × |

Examples 97 to 101 and Comparative Examples 14 and 15

Using the obtained photopolymerization initiator (A) and a known photopolymerization initiator (D), a monofunctional monomer (E), a polyfunctional monomer or oligomer (F), and other components (G) were weighed in the proportion shown in Table 9, and mixed at 25°C for 30 minutes to prepare an active energy ray curable three-dimensional modeling ink composition. Using the obtained three-dimensional modeling ink composition, the three-dimensional modeling curability and curing shrinkage resistance were evaluated. Using the obtained modeling object, the modeling accuracy, strength of the modeling object, heat resistance, and water resistance were evaluated by the following methods, and the results are shown in Table 9.

(5-18) Three-dimensional modeling curability

Using ARM-10 (manufactured by Roland DG Corporation) as a hanging type DLP type vat photopolymerization apparatus, the vat was filled with each of the three-dimensional modeling ink compositions obtained in Examples and Comparative Examples, and irradiated with ultraviolet rays (wavelength: 405 nm, illumination: 0.2 mW/cm$^2$, cumulative amount of light: 5 mJ/cm$^2$) so that the thickness of one layer was 0.15 mm to model a rectangular parallelepiped of 25 × 20 × 1 mm. After the modeling, washing by immersion in isopropanol for 1 minute (twice) was performed to remove the uncured three-dimensional modeling ink composition. Thereafter, by observing the state of the modeling object, the curability was evaluated according to the following criteria.

⊙: A state in which a modeling object was formed and washing with isopropanol was possible while maintaining the shape.
○: A state in which a modeling object was formed, but a part of the modeling object was deformed by washing with isopropanol.
△: A state in which formation of a modeling object was insufficient and the modeling object was damaged by washing with isopropanol.
×: A state in which no modeling object was formed.

(5-19) Curing shrinkage resistance

A 75 μm-thick heavy-release PET film (E7001) was brought into close contact with a horizontally placed glass plate, a spacer with a thickness of 1 mm and an internal size of 60 mm × 100 mm was placed, and each of the three-dimensional modeling ink compositions obtained in Examples and Comparative Examples was filled inside the spacer. Then, light-release PET film (E7002) was further placed thereon, and was cured by irradiation with ultraviolet rays from both sides (wavelength: 405 nm, illumination: 10 mW/cm$^2$, cumulative amount of light: 5,000 mJ/cm$^2$), and the release PET films on both sides were removed to obtain a test piece. The curing shrinkage rate was calculated from the density change of the three-dimensional modeling ink composition and the test piece by the following formula according to JIS K5600 2-4. The density was measured according to JIS K7112 using an electronic densimeter (MDS-300, manufactured by Alfa Mirage Co., Ltd.). From the obtained curing shrinkage rate, the curing shrinkage resistance of the three-dimensional modeling ink composition was evaluated according to the following criteria.

$$\text{Curing shrinkage rate (\%)} = (Ds - Dl)/Dl \times 100\%$$

(In the formula, Ds is the density of the three-dimensional modeling ink composition after curing, and D1 is the density of the three-dimensional modeling ink composition before curing.)

⊙: Curing shrinkage rate was less than 6%.
○: Curing shrinkage rate was 6% or more and less than 7%.
△: Curing shrinkage rate was 7% or more and less than 8%.
×: Curing shrinkage rate was 8% or more.

(5-20) Strength

Test pieces were prepared in the same manner as in the curing shrinkage resistance, 6 sheets were stacked, the Shore D hardness was measured according to JIS K 6253 (Rubber Hardness Test Method), and the strength of the three-dimensional modeling object was evaluated according to the following criteria.

⊙: Shore D hardness was 60 or more.
○: Shore D hardness was 40 or more and less than 60.
×: Shore D hardness was less than 40.

(5-21) Heat resistance

A test piece was prepared in the same manner as in the curing shrinkage resistance, and the glass transition temperature (Tg) of the three-dimensional modeling object was measured with a differential scanning calorimeter (DSC-60 plus, manufactured by Shimadzu Corporation). The heat resistance of the three-dimensional modeling object was evaluated according to the following criteria.

⊙: The Tg of the cured product was 50°C or higher.
○: The Tg of the cured product was 40°C or more and less than 50°C.
×: The Tg of the cured product was less than 40°C.

(5-22) Modeling accuracy

A heavy-release PET film (E7001) was brought into close contact with a horizontally placed glass plate, a spacer with a thickness of 1 mm and an internal size of 10 mm × 10 mm was placed, and each of the three-dimensional modeling ink compositions obtained in Examples and Comparative Examples was filled inside the spacer corresponding to a thickness of 1 mm. Then, the surfaces were smoothed by keeping at 60°C for 30 seconds, and the three-dimensional modeling ink compositions were cured by irradiation with ultraviolet rays (UV-LED lamp: wavelength 405 nm, illumination 10 $mW/cm^2$, cumulative amount of light 1,000 $mJ/cm^2$). Thereafter, the three-dimensional modeling ink composition was filled to a 1 mm thickness and cured 10 times in total to obtain a three-dimensional modeling object of 10 × 10 × 10 mm. The height of the obtained modeling object was measured, and the side surface of the modeling object was visually observed. These results were combined, and the modeling accuracy of the modeling object was evaluated according to the following criteria.

⊙: The height was less than 10 mm ± 0.1 mm, and there was no irregularity on the side surface.
○: The height was 10 mm ± 0.1 mm or more and less than ± 0.2 mm, or there were slight irregularities on the side surface.
△: The height was 10 mm ± 0.2 mm or more and less than ± 0.3 mm, or there were some irregularities on the side surface.
×: The height was 10 mm ± 0.3 mm or more, or there were obvious irregularities on the side surface.

(5-23) Water resistance

A test piece was prepared in the same manner as in the curing shrinkage resistance, and immersed in ion-exchanged water at 25°C for 24 hours to evaluate the water resistance of the three-dimensional modeling according to the following criteria.

$$\text{Water absorption rate (\%)} = (Wb - Wa)/Wa \times 100$$

(In the formula, Wa is the weight of the test piece before immersion in ion-exchanged water, and Wb is the weight of the test piece after immersion in ion-exchanged water.)

⊙: Water absorption rate was less than 0.5%.
○: Water absorption rate was 0.5% or more and less than 1%.
△: Water absorption rate was 1% or more and less than 2%.
×: Water absorption rate was 2% or more.

[Table 9]

| | | Active energy ray curable three-dimensional modeling ink composition (% by mass) | | | | | | Three-dimensional modeling curability | Curing shrinkage resistance | Strength | Heat resistance | Modeling accuracy | Water resistance |
|---|---|---|---|---|---|---|---|---|---|---|---|---|---|
| | | Photo-polymerization initiator (A) | | Mono-functional monomer (E) | | Polyfunctional monomer or oligomer (F) | | | | | | | |
| Examples | 97 | A-19 | 5 | E-3<br>E-5 | 30<br>10 | F-5<br>F-3<br>F-4 | 10<br>15<br>30 | ○ | ○ | ◎ | ○ | ○ | ○ |
| | 98 | A-23 | 30 | E-3<br>E-13 | 30<br>10 | F-9<br>F-16 | 10<br>20 | ◎ | ◎ | ◎ | ◎ | ◎ | ○ |
| | 99 | A-24 | 25 | E-3 | 20 | F-5<br>F-7<br>F-4 | 10<br>5<br>40 | ◎ | ◎ | ◎ | ◎ | ◎ | ○ |
| | 100 | A-25 | 50 | E-4 | 25 | F-15<br>F-10 | 10<br>15 | ◎ | ◎ | ○ | ◎ | ○ | ◎ |
| | 101 | A-31 | 30 | E-3 | 35 | F-10 | 35 | ◎ | ◎ | ◎ | ◎ | ◎ | ○ |
| Comparative Examples | 14 | D-4 | 5 | E-3<br>E-5 | 30<br>10 | F-5<br>F-3<br>F-4 | 10<br>15<br>30 | ◎ | △ | ○ | △ | △ | × |
| | 15 | D-5 | 25 | E-3 | 20 | F-5<br>F-7<br>F-4 | 10<br>5<br>40 | △ | ○ | △ | △ | △ | △ |

Examples 102 to 106 and Comparative Examples 16 and 17

Using the obtained photopolymerization initiator (A) and a known photopolymerization initiator (D), a monofunctional monomer (E), a polyfunctional monomer or oligomer (F), and other components (G) were weighed in the proportion shown in Table 10, and mixed at 25°C for 30 minutes to prepare a composition for an active energy ray curable nail cosmetic. Using the obtained nail cosmetic composition, the curability of the nail cosmetic composition was evaluated in the same manner as in the curability evaluation under conditions without oxygen inhibition described in (5-2) above (LED 405 nm). The adhesion, surface hardness, surface gloss, and removability of the nail cosmetic composition were evaluated by the following methods, and the results are shown in Table 10.

(5-24) Adhesion

The curable nail cosmetic composition was applied onto a nylon 6 test piece and irradiated for 3 minutes using a gel nail dedicated UV-LED lamp (manufactured by Beauty Nailer, wavelength 405 nm, output power 48 W) to prepare a cured film. Using the obtained cured film, the adhesion of the nail cosmetic composition was evaluated according to the following criteria in the same manner as in the adhesion evaluation of the coating composition according to JIS K 5600. The greater the number of squares remaining on the test piece, the higher the adhesion.

⊙: The number of remaining squares was 100.
○: The number of remaining squares was 90 to 99.
△: The number of remaining squares was 60 to 89.
×: The number of remaining squares was less than 60.

(5-25) Surface hardness

A cured film was prepared in the same manner as in the adhesion evaluation, and the surface of the film was

pulled with a pencil of HB hardness by pressing a load of 750 g at an angle of 45°, and the occurrence of peeling and the presence or absence of scratches were visually confirmed, and the surface hardness was evaluated according to the following criteria. The fewer the occurrence of scratches or peeling, the higher the surface hardness.

○: No scratches or peeling occurred. Surface hardness was greater than pencil hardness HB.
△: Peeling did not occur, but scratches occurred.
×: Peeling occurred.

(5-26) Surface glossiness

A cured film was prepared in the same manner as in the adhesion evaluation, and allowed to stand for 24 hours in a thermo-hygrostat bath at a temperature of 40°C and a relative humidity of 50%, and then the gloss of the surface of the film was visually observed, and the surface gloss of the cured film was evaluated according to the following criteria.

○: The gloss was confirmed.
△: Although reflection of light could be confirmed, cloudiness was observed.
×: Reflection of light could not be confirmed, and there was no gloss.

(5-27) Removability

A cured film having a size of 10 mm × 10 mm was prepared on a test piece in the same manner as in the adhesion evaluation, and the test piece was covered with absorbent cotton immersed in acetone for 5 minutes. Thereafter, the absorbent cotton was removed, and the cured film was rubbed 10 times with a cotton swab. The state in which the cured film was peeled off from the test piece was observed, and the removability of the cured film was evaluated according to the following criteria.

○: The cured film was peeled off without remaining.
△: The cured film was peeled off, but a part of the cured film remained.
×: A large part of the cured film remained.

[Table 10]

| | | Composition for active energy ray curable nail cosmetic (% by mass) | | | | | | | | Curability | Adhesion | Surface hardness | Surface glossiness | Removability |
|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | | | | |
| Examples | 102 | A-11 | 10 | E-2<br>E-3<br>E-7 | 20<br>20<br>10 | F-16 | 35 | G-2 | 5 | ○ | ○ | ○ | ○ | ○ |
| | 103 | A-20 | 10 | E-3<br>E-14 | 20<br>20 | F-3 | 45 | G-2 | 5 | ◎ | ◎ | ○ | ○ | ○ |
| | 104 | A-23 | 40 | E-3<br>E-14 | 20<br>15 | F-1 | 15 | G-4 | 10 | ◎ | ◎ | ○ | ○ | ○ |
| | 105 | A-32 | 20 | E-2 | 25 | F-3<br>F-10 | 30<br>20 | G-2 | 5 | ○ | ○ | ○ | ○ | ○ |
| | 106 | A-33 | 45 | E-2<br>E-3<br>E-7 | 20<br>20<br>10 | - | - | G-2 | 5 | ◎ | ◎ | ○ | ○ | ○ |
| Comparative Examples | 16 | D-1 | 10 | E-2<br>E-3<br>E-7 | 20<br>20<br>10 | F-16 | 35 | G-2 | 5 | △ | △ | × | △ | △ |
| | 17 | D-5 | 20 | E-2 | 25 | F-3<br>F-10 | 30<br>20 | G-2 | 5 | △ | ○ | △ | × | × |

Examples 107 to 112 and Comparative Examples 18 and 19

Using the obtained photopolymerization initiator (A) and a known photopolymerization initiator (D), a monofunctional monomer (E), a polyfunctional monomer or oligomer (F), and other components (G) were weighed in the proportion shown in Table 11, and mixed at 25°C for 30 minutes to prepare an active energy ray curable dental material composition. Using the obtained dental material composition, the solubility (dispersibility), storage stability, and curability (in the same manner as in the evaluation of curability under conditions without oxygen inhibition in (5-2) above, LED 405 nm) were evaluated, and the hardness, surface smoothness, and adhesive strength of the obtained cured product were evaluated according to the following criteria, and the results are shown in Table 11.

(5-28) Solubility (dispersibility)

The state of the curable dental material composition was visually observed, and the solubility or dispersibility was evaluated according to the following criteria.

○: The composition was homogeneous.
△: The composition was found to be slightly heterogeneous.
×: The composition was heterogeneous.

(5-29) Storage stability

The curable dental material composition was placed in a light-blocking screw tube. After the lid was closed, the screw tube was stored under two conditions at 40°C for 1 month and at 80°C for 2 weeks. The dissolved or dispersed state of the composition after storage was confirmed to evaluate the storage stability of the composition according to the following criteria.

○: There was no change in state after storage for both conditions at 40°C for 1 month and at 80°C for 2 weeks.
△: Changes in state after storage were confirmed at either one condition at 40°C for 1 month or at 80°C for 2 weeks.

×: Changes in state after storage were confirmed under both conditions at 40°C for 1 month and at 80°C for 2 weeks.

(5-30) Hardness

The surface of the cured product obtained in the curability evaluation was buffed, and the Knoop hardness was measured at a temperature of 23°C using a microhardness meter manufactured by Matsuzawa Seiki under a load of 10 g for 20 seconds, and the hardness of the cured product was evaluated according to the following criteria.

⊙: Knoop hardness was 200 KHN or more (equivalent to permanent tooth enamel).
○: Knoop hardness was 70 KHN or more and less than 200 KHN (equivalent to dentin).
△: Knoop hardness was less than 70 KHN.
×: Measurement could not be performed because it was not cured.

(5-31) Surface smoothness

The surface smoothness and gloss of the cured product obtained in the curability evaluation were visually observed and evaluated according to the following criteria.

⊙: The surface was smooth and shiny.
○: The surface was almost smooth, and slight cloudiness or slight irregularities were observed.
△: The surface was entirely cloudy, and some irregularities or some grains were observed.
×: The surface was entirely cloudy and covered with grains.

(5-32) Adhesive strength

The bovine lower forehead front teeth were polished with No. 1000 water-resistant abrasive paper under water injection, a flat dentine surface for bonding was cut out, dried by blowing compressed air for 10 seconds, and a tape having a hole with a diametral 3 mm was attached to set an adhesive surface. Thereafter, an adhesive test piece was prepared by a known method (see the method described in JP 2010-208964 A). The adhesive test piece was immersed in water at 37°C for 24 hours, and then the tensile adhesive strength was measured with an Instron universal tester (crosshead speed rate: 2 mm/min) to determine the adhesive strength between enamel and dentin, and the adhesive strength was evaluated according to the following criteria.

⊙: Both enamel adhesive strength and dentin adhesive strength were 20 MPa or more.
○: Only one of the adhesive strength of enamel or dentin was 20 MPa or more.
△: Both enamel adhesive strength and dentin adhesive strength were 7 MPa or more.
×: Both enamel adhesive strength and dentin adhesive strength were less than 7 MPa.

[Table 11]

| | | Active energy ray curable dental material composition (% by mass) | | | | | | | | Solubility (dispersibility) | Storage stability | Curability | Hardness | Surface smoothness | Adhesive strength |
|---|---|---|---|---|---|---|---|---|---|---|---|---|---|---|---|
| | | Photo-polymerization initiator (A) | | Mono-functional monomer (E) | | Polyfunctional monomer or oligomer (F) | | Other components (G) | | | | | | | |
| Examples | 107 | A-16 | 20 | E-10<br>E-11<br>E-15 | 30<br>10<br>5 | F-12 | 25 | G-4 | 10 | ○ | ○ | ◎ | ◎ | ○ | ◎ |
| | 108 | A-17 | 10 | E-10<br>E-14 | 30<br>10 | F-16 | 40 | G-8 | 10 | ○ | ○ | ○ | ○ | ◎ | ○ |
| | 109 | A-20<br>A-22 | 5<br>5 | E-1 | 20 | F-8<br>F-17 | 30<br>30 | G-8 | 10 | ○ | ○ | ○ | ◎ | ○ | ○ |
| | 110 | A-23 | 30 | E-3<br>E-13 | 15<br>10 | F-14 | 30 | G-4<br>G-8 | 5<br>10 | ○ | ○ | ◎ | ◎ | ◎ | ○ |
| | 111 | A-24 | 30 | E-1<br>E-15 | 30<br>5 | F-12 | 30 | G-8 | 5 | ○ | ○ | ◎ | ○ | ○ | ◎ |
| | 112 | A-25 | 10 | E-8<br>E-11<br>E-15 | 35<br>10<br>10 | F-13 | 30 | G-4 | 5 | ○ | ○ | ◎ | ◎ | ○ | ◎ |
| Comparative Examples | 18 | D-5 | 10 | E-1 | 20 | F-8<br>F-17 | 30<br>30 | G-8 | 10 | × | × | △ | △ | × | △ |
| | 19 | D-6 | 10 | E-8<br>E-11<br>E-15 | 35<br>10<br>10 | F-13 | 30 | G-4 | 5 | △ | △ | △ | ○ | △ | ○ |

Examples 113 to 118 and Comparative Examples 20 and 21

Using the obtained photopolymerization initiator (A) and a known photopolymerization initiator (D), a polyfunctional monomer or oligomer (F), other components (G), and an organic solvent (H) were weighed in the proportion shown in Table 12, and mixed at 25°C for 30 minutes to prepare an active energy ray curable photosensitive composition. Using the obtained photosensitive composition, photosensitive resins for evaluation were prepared in (5-33) Production 1 of the photosensitive resin below in Examples 113 to 115 and Comparative Example 20, and in (5-34) Production 2 of the photosensitive resin below in Examples 116 to 118 and Comparative Example 21, and the sensitivity, patternability, and storage stability of the photosensitive composition were evaluated, and the results are shown in Table 12.

(5-33) Production 1 of photosensitive resin

Using the active energy ray curable photosensitive compositions of Examples 113 to 115 and Comparative Example 20, the compositions were applied to a PET film (E5100, corona-treated surface) by a rotary coating machine so as to have a film thickness of 15 μm, and dried in an oven at 80°C for 3 minutes. Thereafter, irradiation with ultraviolet rays (wavelength: 405 nm, illumination: 0.5 mW/cm$^2$, cumulative amount of light: 90 mJ/cm$^2$) was performed for 3 minutes using a negative photomask. Next, the negative photomask was removed, and the unexposed portion was removed using cyclopentanone to obtain a photosensitive resin cured product for evaluation.

(5-34) Production 2 of photosensitive resin

Using the active energy ray curable photosensitive compositions of Examples 116 to 118 and Comparative Example 21, the compositions were applied to a PET film (E5100, corona-treated surface) by a rotary coating machine so as to have a film thickness of 15 μm, and dried in an oven at 40°C for 30 minutes. Thereafter, irradiation with ultraviolet rays (wavelength: 405 nm, illumination: 0.5 mW/cm$^2$, cumulative amount of light: 90 mJ/cm$^2$) was performed for 3 minutes using a negative photomask, the negative photomask was then removed, and the unexposed portion was removed using

cyclopentanone. Next, heat treatment was performed in an oven at 130°C for 30 minutes to obtain a photosensitive resin for evaluation.

(5-35) Sensitivity

The obtained photosensitive resin for evaluation was touched with a hand to confirm the presence or absence of stickiness or an uncured component, and the curability of the photosensitive composition was evaluated according to the following criteria.

⊙: There was no stickiness.
○: There was slight stickiness, but no finger mark remained on the surface.
△: There was stickiness, and a finger mark remained on the surface.
×: Stickiness was severe, and a finger stuck to the surface.

(5-36) Patternability

The patternability of the photosensitive resin for evaluation was evaluated according to the following criteria.

⊙: There was no distortion in the pattern, and no chipping of the edge portion was observed.
○: There was no distortion in the pattern, and the edge portion was slightly chipped.
△: There was no distortion in the pattern, and chipping of the edge portion was observed.
×: There was distortion in the pattern.

(5-37) Storage stability

The obtained photosensitive resin for evaluation was allowed to stand in a thermo-hygrostat bath at a temperature of 40°C and a relative humidity of 50% for 168 hours, the presence or absence of bleed-out on the surface of the test piece was visually observed, and the storage stability was evaluated according to the following criteria. The smaller the bleed-out, the higher the storage stability.

⊙: No bleed-out was observed.
○: Bleed-out was slightly observed.
×: Bleed-out was severely observed.

[Table 12]

| | | Active energy ray curable photosensitive composition (% by mass) | | | | | | | | Sensitivity | Patternability | Storage stability |
|---|---|---|---|---|---|---|---|---|---|---|---|---|
| | | Photo-polymerization initiator (A) | | Polyfunctional monomer or oligomer (F) | | Other components (G) | | Organic solvent (H) | | | | |
| Examples | 113 | A-3 | 20 | F-15 | 25 | G-9<br>G-10 | 20<br>5 | H-4 | 30 | ○ | ○ | ○ |
| | 114 | A-16 | 10 | F-11 | 35 | G-9<br>G-10 | 19<br>1 | H-3 | 35 | ○ | ○ | ◎ |
| | 115 | A-20 | 5 | F-9 | 20 | G-9<br>G-10 | 10<br>5 | H-2 | 60 | ◎ | ◎ | ◎ |
| | 116 | A-23 | 50 | - | | G-9 | 10 | H-2 | 40 | ◎ | ○ | ◎ |
| | 117 | A-26 | 15 | F-7 | 25 | G-9 | 5 | H-1 | 55 | ○ | ○ | ◎ |
| | 118 | A-31 | 30 | F-7 | 15 | G-9 | 20 | H-1<br>H-2 | 20<br>15 | ◎ | ◎ | ○ |
| Comparative Examples | 20 | D-2 | 10 | F-9 | 15 | G-9<br>G-10 | 10<br>5 | H-2 | 60 | ○ | △ | × |
| | 21 | D-15 | 15 | F-7 | 25 | G-9 | 5 | H-1 | 55 | ○ | ○ | × |

It is clear from the results of Tables 4 to 12, that the photopolymerization initiator (A) of the present disclosure (Examples 1 to 33) had good compatibility with a general-purpose monofunctional monomer or polyfunctional monomer or oligomer, and high photo initiating property with various active energy rays from a short wavelength to a long wavelength. In addition, the active energy ray curable composition containing the photopolymerization initiator (A) had high curability. Both the obtained curable composition and the cured product were excellent in transparency. The photopolymerization initiator (A) is hardly affected by inhibition by oxygen, and both an initiation reaction by a long-wavelength light in air and a photopolymerization reaction can proceed at a sufficient rate. The curable composition containing the photopolymerization initiator (A) (Examples 34 to 72) had high curability even under oxygen interruption or irradiation with long-wavelength light in air. The active energy ray curable composition adjusted for various applications (Examples 73 to 118) were cured to obtain various forms cured products, which exhibit good adhesion, pressure-sensitive adhesiveness, and adhesiveness, and have high physical properties such as hardness and strength. Furthermore, the cured product obtained by curing had very little odor, bleed-out, outgassing, yellowing over time, deterioration, and the like, and the obtained cured product had high yellowing resistance, water resistance, durability, heat resistance, and sealing ability. On the other hand, the composition containing a known photopolymerization initiator (Comparative Examples 1 to 21) had low curability with respect to long-wavelength light; low adhesion, pressure-sensitive adhesiveness, adhesiveness, and physical properties such as hardness and strength of the obtained cured product; and low yellowing resistance, water resistance, durability, and heat resistance. Therefore, the photopolymerization initiator of the present disclosure and the active energy ray curable composition containing the photopolymerization initiator can be suitably used for various applications.

The present disclosure includes the following contents.

(1) A photopolymerization initiator having one or more benzophenone groups and one or more saturated or unsaturated 5 or more-membered cyclic substituents having heteroatoms in a molecule, wherein one or more saturated or unsaturated 5 or more-membered cyclic substituents having heteroatoms are bonded to one or more carbon atoms of an aryl group of one or more benzophenone groups through a carboxylic acid ester group or a carboxylic acid amide group.

(2) The photopolymerization initiator according (1), having one or more ethylenically unsaturated groups of one or more kinds selected from one or more (meth)acrylamide groups, (meth)acrylate groups, vinyl groups, vinyl ether groups, alkyl vinyl ether groups, allyl groups, (meth)allyl ether groups, styryl groups, and maleimide groups in a

molecule.
(3) The photopolymerization initiator according to (1) or (2), wherein the one or more cyclic substituents are one or more groups selected from a piperidine group, a pyrrolidine group, a piperazine group, a pyridine group, a morpholine group, a tetrahydrofuran group, a hydrofuran group, a crown ether group, and a tetrahydrothiopyran group.
(4) The photopolymerization initiator according to claim 1 or 2, wherein the one or more cyclic substituents are one or more groups selected from a piperidine group, a pyrrolidine group, a piperazine group, a pyridine group, a morpholine group, a tetrahydrofuran group, a hydrofuran group, a crown ether group, and a tetrahydrothiopyran group.
The photopolymerization initiator according to any one of claims 1 to 3, having one or more groups selected from one or more urethane groups, urea groups, ester groups, thioester groups, amide groups, and imide groups in a molecule.
(5) The photopolymerization initiator according to any one of (1) to (4), having a structure represented by general formula (1),

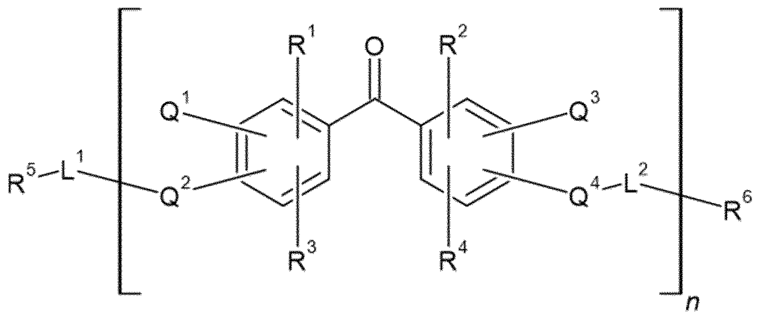

general formula (1)

wherein $Q^1$ and $Q^3$ are each independently a hydrogen atom or a monovalent organic group represented by general formula (2) or general formula (3), and any one or more of $Q^1$ and $Q^3$ are a monovalent organic group having one or more saturated or unsaturated 5 or more-membered cyclic substituents having heteroatoms,
$Q^2$ and $Q^4$ are each independently a divalent organic group represented by general formula (4) or general formula (5),
$L^1$ and $L^2$ are each independently a direct bond or a divalent organic group containing one or more of a urethane group, a urea group, an ester group, an amide group, and an imide group, and $Q^2$-$L^1$-$R^5$ and $Q^4$-$L^2$-$R^6$ may be a hydrogen atom, except a case where $Q^2$-$L^1$-$R^5$ and $Q^4$-$L^2$-$R^6$ are simultaneously a hydrogen atom together with $Q^1$ and $Q^3$,

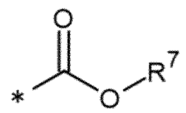

general formula (2)

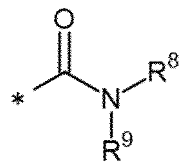

general formula (3)

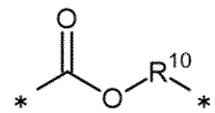

general formula (4)

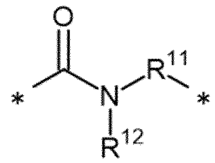

general formula (5)

$R^1$ to $R^9$ and $R^{12}$ each independently represent a hydrogen atom or a chain or cyclic, saturated or unsaturated monovalent hydrocarbon group having 1 to 36 carbon atoms in which one or more hydrogen atoms may be

substituted with a hydroxyl group, an amine group, a thiol group, or a halogen group and one or more carbon atoms may be substituted with an ether group, an amino group, a thioether group, or a thioester group,
$R^{10}$ and $R^{11}$ each independently represent a direct bond or a chain or cyclic, saturated or unsaturated divalent hydrocarbon group having 1 to 36 carbon atoms in which one or more hydrogen atoms may be substituted with a hydroxyl group, an amine group, a thiol group, or a halogen group and one or more carbon atoms may be substituted with an ether group, an amino group, a thioether group, or a thioester group,
$R^8$ and $R^9$ may be taken together with a nitrogen atom carrying $R^8$ and $R^9$ to form a structure of a 5-membered to 7-membered cyclic substituent, except a case where $R^8$ and $R^9$ are simultaneously a hydrogen atom,
$R^{10}$ and $R^{11}$ may be taken together with a nitrogen atom carrying $R^{10}$ and $R^{11}$ to form a structure of a 5-membered to 7-membered cyclic substituent,
any one or more of $R^5$, $R^6$, $R^7$, $R^8$, $R^9$, $R^{10}$, $R^{11}$, and $R^{12}$ have a saturated or unsaturated 5 or more-membered cyclic substituent having heteroatoms, and
n is an integer of 1 to 100.

(6) An active energy ray curable composition containing the photopolymerization initiator according to any one of (1) to (5).
(7) An active energy ray curable ink composition containing the photopolymerization initiator according to any one of (1) to (5).
(8) An active energy ray curable pressure-sensitive adhesive composition containing the photopolymerization initiator according to any one of (1) to (5).
(9) An active energy ray curable adhesive composition containing the photopolymerization initiator according to any one of (1) to (5).
(10) An active energy ray curable coating composition containing the photopolymerization initiator according to any one of claims 1 to 5.
(11) An active energy ray curable sealant composition containing the photopolymerization initiator according to any one of (1) to (5).
(12) An active energy ray curable inkjet ink containing the photopolymerization initiator according to any one of (1) to (5).
(13) An active energy ray curable three-dimensional modeling ink containing the photopolymerization initiator according to any one of (1) to (5).
(14) An active energy ray curable nail cosmetic composition containing the photopolymerization initiator according to any one of (1) to (5).
(15) An active energy ray curable dental material composition containing the photopolymerization initiator according to any one of (1) to (5).
(16) An active energy ray curable photosensitive composition containing the photopolymerization initiator according to any one of (1) to (5).
(17) An active energy ray curable hydrogel composition containing the photopolymerization initiator according to any one of (1) to (5).
(18) An active energy ray curable intraocular implant material composition containing the photopolymerization initiator according to any one of (1) to (5).
(19) An active energy ray curable skin pressure-sensitive adhesive composition containing the photopolymerization initiator according to any one of (1) to (5).
(20) An active energy ray curable biological adhesive composition containing the photopolymerization initiator according to any one of (1) to (5).
(21) An active energy ray curable automobile coating-repairing composition containing the photopolymerization initiator according to any one of (1) to (5).

INDUSTRIAL APPLICABILITY

As described above, the photopolymerization initiator (A) of the present disclosure has high photoinitiating property, and light sources having various wavelengths such as UV-LED lamps with wavelengths of 360 nm to 420 nm can be used, and initiation of photopolymerization and a curing reaction of an active energy ray in the presence of oxygen can be performed. In addition, there is no generation of decomposition products in a photoinitiation reaction and a photopolymerization reaction (curing), safety is high, and the resulting cured product has very little odor, bleed-out, yellowing over time, deterioration, and the like. **In** particular, when an ethylenically unsaturated group is contained, the photopolymerization initiator becomes a constituent unit of a cured product after curing, and a cured product having good physical properties and durability is obtained. By combining a photopolymerization initiator of the present invention with various compounds containing an unsaturated group, it is possible to produce active energy ray curable compositions

used for various applications, and it is possible to impart various physical properties such as high adhesion, surface hardness, yellowing resistance, and water resistance. The photopolymerization initiator of the present invention can be suitably used as an active energy ray curable composition, an active energy ray curable ink composition, an active energy ray curable pressure-sensitive adhesive composition, an active energy ray curable adhesive composition, an active energy ray curable coating composition, an active energy ray curable sealant composition, an active energy ray curable inkjet ink composition, an active energy ray curable three-dimensional modeling ink composition, an active energy ray curable nail cosmetic composition, an active energy ray curable dental composition, an active energy ray curable type photosensitive composition, an active energy ray curable hydrogel composition, an active energy ray curable intraocular implant material composition, an active energy ray curable skin pressure-sensitive adhesive composition, an active energy ray curable biological adhesive composition, an active energy ray curable automobile coating-repairing composition, or the like.

## Claims

1. A photopolymerization initiator having one or more benzophenone groups and one or more saturated or unsaturated 5 or more-membered cyclic substituents having heteroatoms in a molecule, wherein one or more saturated or unsaturated 5 or more-membered cyclic substituents having heteroatoms are bonded to one or more carbon atoms of an aryl group of one or more benzophenone groups through a carboxylic acid ester group or a carboxylic acid amide group.

2. The photopolymerization initiator according claim 1, having one or more ethylenically unsaturated groups of one or more kinds selected from one or more (meth)acrylamide groups, (meth)acrylate groups, vinyl groups, vinyl ether groups, alkyl vinyl ether groups, allyl groups, (meth)allyl ether groups, styryl groups, and maleimide groups in a molecule.

3. The photopolymerization initiator according to claim 1 or 2, wherein the one or more cyclic substituents are one or more groups selected from a piperidine group, a pyrrolidine group, a piperazine group, a pyridine group, a morpholine group, a tetrahydrofuran group, a hydrofuran group, a crown ether group, and a tetrahydrothiopyran group.

4. The photopolymerization initiator according to any one of claims 1 to 3, having one or more groups selected from one or more urethane groups, urea groups, ester groups, thioester groups, amide groups, and imide groups in a molecule.

5. The photopolymerization initiator according to any one of claims 1 to 4, having a structure represented by general formula (1),

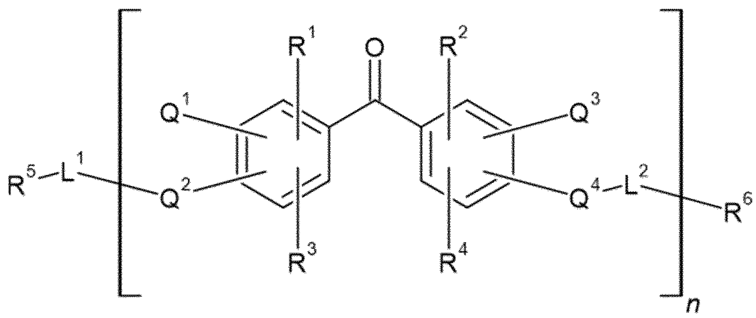

general formula (1)

wherein $Q^1$ and $Q^3$ are each independently a hydrogen atom or a monovalent organic group represented by general formula (2) or general formula (3), and any one or more of $Q^1$ and $Q^3$ are a monovalent organic group having one or more saturated or unsaturated 5 or more-membered cyclic substituents having heteroatoms,
$Q^2$ and $Q^4$ are each independently a divalent organic group represented by general formula (4) or general formula (5),
$L^1$ and $L^2$ are each independently a direct bond or a divalent organic group containing one or more of a urethane group, a urea group, an ester group, an amide group, and an imide group, and $Q^2$-$L^1$-$R^5$ and $Q^4$-$L^2$-$R^6$ may be a hydrogen atom, except a case where $Q^2$-$L^1$-$R^5$ and $Q^4$-$L^2$-$R^6$ are simultaneously a hydrogen atom together with $Q^1$ and $Q^3$,

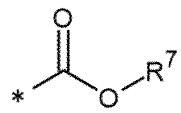
general formula (2)

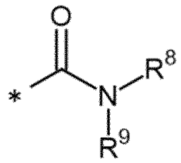
general formula (3)

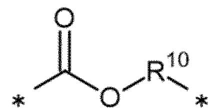
general formula (4)

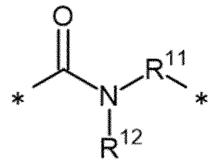
general formula (5)

$R^1$ to $R^9$ and $R^{12}$ each independently represent a hydrogen atom or a chain or cyclic, saturated or unsaturated monovalent hydrocarbon group having 1 to 36 carbon atoms in which one or more hydrogen atoms may be substituted with a hydroxyl group, an amine group, a thiol group, or a halogen group and one or more carbon atoms may be substituted with an ether group, an amino group, a thioether group, or a thioester group,
$R^{10}$ and $R^{11}$ each independently represent a direct bond or a chain or cyclic, saturated or unsaturated divalent hydrocarbon group having 1 to 36 carbon atoms in which one or more hydrogen atoms may be substituted with a hydroxyl group, an amine group, a thiol group, or a halogen group and one or more carbon atoms may be substituted with an ether group, an amino group, a thioether group, or a thioester group,
$R^8$ and $R^9$ may be taken together with a nitrogen atom carrying $R^8$ and $R^9$ to form a structure of a 5-membered to 7-membered cyclic substituent, except a case where $R^8$ and $R^9$ are simultaneously a hydrogen atom,
$R^{10}$ and $R^{11}$ may be taken together with a nitrogen atom carrying $R^{10}$ and $R^{11}$ to form a structure of a 5-membered to 7-membered cyclic substituent,
any one or more of $R^5$, $R^6$, $R^7$, $R^8$, $R^9$, $R^{10}$, $R^{11}$, and $R^{12}$ have a saturated or unsaturated 5 or more-membered cyclic substituent having heteroatoms, and
n is an integer of 1 to 100.

**6.** An active energy ray curable composition comprising the photopolymerization initiator according to any one of claims 1 to 5.

**7.** An active energy ray curable ink composition comprising the photopolymerization initiator according to any one of claims 1 to 5.

**8.** An active energy ray curable pressure-sensitive adhesive composition comprising the photopolymerization initiator according to any one of claims 1 to 5.

**9.** An active energy ray curable adhesive composition comprising the photopolymerization initiator according to any one of claims 1 to 5.

**10.** An active energy ray curable coating composition comprising the photopolymerization initiator according to any one of claims 1 to 5.

**11.** An active energy ray curable sealant composition comprising the photopolymerization initiator according to any one of claims 1 to 5.

**12.** An active energy ray curable inkjet ink comprising the photopolymerization initiator according to any one of claims 1 to

5.

**13.** An active energy ray curable three-dimensional modeling ink comprising the photopolymerization initiator according to any one of claims 1 to 5.

**14.** An active energy ray curable nail cosmetic composition comprising the photopolymerization initiator according to any one of claims 1 to 5.

**15.** An active energy ray curable dental material composition comprising the photopolymerization initiator according to any one of claims 1 to 5.

**16.** An active energy ray curable photosensitive composition comprising the photopolymerization initiator according to any one of claims 1 to 5.

**INTERNATIONAL SEARCH REPORT**

International application No.
**PCT/JP2023/037865**

**A. CLASSIFICATION OF SUBJECT MATTER**

***C08F 2/50***(2006.01)i; ***A61K 6/62***(2020.01)i; ***A61K 8/37***(2006.01)i; ***A61Q 3/02***(2006.01)i; ***B33Y 70/00***(2020.01)i; ***B33Y 80/00***(2015.01)i; ***C08G 18/30***(2006.01)i; ***C08G 18/42***(2006.01)i; ***C08G 18/60***(2006.01)i; ***C09D 11/30***(2014.01)i
FI: C08F2/50; A61K6/62; A61K8/37; A61Q3/02; B33Y70/00; B33Y80/00; C08G18/30; C08G18/42 008; C08G18/60; C09D11/30

According to International Patent Classification (IPC) or to both national classification and IPC

**B. FIELDS SEARCHED**

Minimum documentation searched (classification system followed by classification symbols)

C08F2/50; A61K6/62; A61K8/37; A61Q3/02; B33Y70/00; B33Y80/00; C08G18/30; C08G18/42; C08G18/60; C09D11/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | CN 111138290 A (SHANXI UNIVERSITY) 12 May 2020 (2020-05-12)<br>claims, examples | 1, 4-16<br>2-3 |
| X<br>A | CN 103926794 A (CHANGZHOU TRONLY NEW ELECTRONIC MATERIALS CO., LTD.) 16 July 2014 (2014-07-16)<br>claims, paragraph [0028] | 1, 3-16<br>2 |
| A | CN 111205203 A (SHANXI UNIVERSITY) 29 May 2020 (2020-05-29)<br>claims, examples | 1-16 |
| P, A | WO 2023/074620 A1 (KJ CHEMICALS CORP) 04 May 2023 (2023-05-04)<br>claims, examples | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **26 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/037865**

| Patent document cited in search report | Publication date (day/month/year) | Patent family member(s) | Publication date (day/month/year) |
|---|---|---|---|
| CN 111138290 A | 12 May 2020 | (Family: none) | |
| CN 103926794 A | 16 July 2014 | (Family: none) | |
| CN 111205203 A | 29 May 2020 | (Family: none) | |
| WO 2023/074620 A1 | 04 May 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010208964 A